# EUROPEAN PATENT SPECIFICATION

(11) **EP 2 585 210 B1**
(45) Date of publication and mention of the grant of the patent: **02.03.2016**
(21) Application number: 11730620.9
(22) Date of filing: 28.06.2011
(51) Int. Cl.: B01J 13/18

(54) **A MICELLULAR COMBINATION COMPRISING A NANOPARTICLE AND A PLURALITY OF SURFMER LIGANDS**
MIZELLARE KOMBINATION AUS EINEM NANOPARTIKEL UND MEHREREN SURFMER-LIGANDEN
COMBINAISON MICELLAIRE COMPRENANT UNE NANOPARTICULE ET UNE PLURALITÉ DE LIGANDS TENSIOACTIFS POLYMÉRISABLES (SURFOMÈRES)

(30) Priority: 28.06.2010 GB 201010831
(43) Date of publication of application: 01.05.2013
(73) Proprietor: Centrum für Angewandte Nanotechnologie (CAN) GmbH, 20146 Hamburg (DE)
(72) Inventor: PÖSELT, Elmar, 22359 Hamburg (DE); KLOUST, Hauke, 22549 Hamburg (DE); WELLER, Horst, 22393 Hamburg (DE); SCHMIDTKE, Christian, 20253 Hamburg (DE); FUHRMANN, Christian, 22069 Hamburg (DE); KAPPEN, Sascha, 22303 Hamburg (DE); PAUER, Werner, 22926 Hamburg (DE); MORITZ, Hans-Ulrich, 21227 Bendestorf (DE)
(74) Representative: Hoffmann Eitle
(86) International application number: PCT/EP2011/060852
(87) International publication number: WO 2012/001012

(56) References cited:
- WO-A1-2009/021286
- WO-A1-2009/025623
- WO-A2-2008/137733
- US-A1- 2004 033 345
- US-A1- 2005 142 567
- US-A1- 2008 107 911
- CHANTAL PAQUET ET AL: "Nanobeads Highly Loaded with Superparamagnetic Nanoparticles Prepared by Emulsification and Seeded-Emulsion Polymerization", LANGMUIR, vol. 26, no. 8, 20 April 2010 (2010-04-20) , pages 5388-5396, XP055046624, ISSN: 0743-7463, DOI: 10.1021/la903815t cited in the application

## Description

### Cross reference to related applications

The present application claims benefit and priority of UK patent application No. GB 1010831.4 filed on 28 June 2010.

### Field of invention

The field of the present invention relates to the stabilisation of nanoparticles in aqueous dispersion and, in particular, the stabilisation of nanoparticles by encapsulating the nanoparticles in a micellular combination. The field of the present invention further relates to a micellular combination of nanoparticles and a method of manufacture of the micellular combination and uses thereof.

### Background of the invention

Inorganic nanoparticles are used in a wide range of applications, for example electronics, biological applications and in medical imaging. The inorganic nanoparticles can be, for example, SPIONs (supra paramagnetic iron oxide nanoparticles), semiconducting nanoparticles (such as quantum dots) or metal nanoparticles.

The number of applications that utilise the full potential of the inorganic nanoparticles is limited by the hydrophobic nature of the inorganic nanoparticles. The hydrophobic nature of the inorganic nanoparticles leads to an inherently poor solubility of the inorganic nanoparticles in aqueous media. Methods to overcome the inherently poor solubility of the inorganic nanoparticles in aqueous media, rely on coating of the nanoparticles with polymeric ligands or other stabilising ligands, which act as interface between the hydrophobic particle surface and the hydrophilic environment, e.g. as described by R. Jin et al. in Angewandte Chemie, International Edition 2008, 47, 6750-6750. The attachment of these stabilising ligands to the nanoparticle surface can be achieved by leveraging on different physical effects, e.g. physisorption, electrostatic attraction, or van der Waals interactions. For instance, the stabilising ligands can be equipped with a suitable terminal anchor group (for example dihydrolipoic acid and derivatives thereof), which displays high affinity to the surface of the inorganic nanoparticle. However, known issues of these methods are that the stabilising ligands often display liabilities, depending on different factors e.g. ionic strength, pH value, protein concentration of the aqueous media and the like. For instance, a gradual detachment over the time from the nanoparticle surface may result in undesired effects, e.g. uncontrolled aggregation, precipitation, change of physical properties, e.g. fluorescence, magnetic properties or change of biological properties, e.g. toxicity etc.. Furthermore, the detachment of the ligand can also negatively impact the performance of the inorganic nanoparticles by removing surface atoms from the inorganic nanoparticles.

Some attempts have been made, to crosslink the stabilising ligands in order to minimize ligand detachment from the nanoparticle surface (US 7,754,329B2; WO 2010100108A1). However, these methods are restricted to specially designed crosslinkable ligands. Furthermore, the thickness of the crosslinked shell, and thus the stability of the final water-solubilized nanoparticle, is defined through the choice of the ligands and cannot be tuned by the crosslinking process.

The properties of inorganic nanoparticles can be modified by assembling single inorganic nanoparticles to aggregates containing a multitude of nanoparticles, and can be controlled depending on the cluster rate of the nanoparticles. E.g. in MRI clustered SPION show higher r₂ relaxivities than non-clustered, e.g. as described by Matsumoto et al. (Magnetic Resonance Imaging, 2008, 26, 994-998).

Concerning quantum dots, radiationless energy transfer can occur in quantum dot clusters, thus reducing the fluorescence quantum yield. Depending on the application, different cluster sizes are required; therefore the technique transferring the nanoparticles into water should allow tuning the size of the clusters from single nanoparticles to large clusters.

### Prior art

Microemulsion polymerisation is a technique, were nanoscale organic polymer particles with a narrow size distribution can be produced. By these methods, a polymerisable monomer is transferred to an aqueous solution containing a surfactant. In the aqueous phase, due to its limited solubility most of the monomer forms macroscopic droplets, whereas the surfactant forms nanoscale micelles. A fraction of solubilised monomer successively diffuses from the monomer droplets into the surfactant micelles wherein it polymerises, thus forming uniform latex particles. (G. Becker, Polystyrol, Hanser München, 1996). Albeit these methods are very compelling e.g. because micellular emulsions of infinite thermodynamic stability can be generated without application of energy, hitherto were not applicable to the encapsulation of nanoparticles, simply because - unlike the monomer - diffusion of the nanoparticles through the water phase into the micelles is impossible. Rather, the inorganic nanoparticles would stay in the depleting monomer droplets and finally conglomerate to deposits of undefined structure in an uncontrolled manner.

In contrast, miniemulsion polymerisation techniques, rather than microemulsion polymerisation technique require the application of energy (e.g., ultrasonication, Ultra-Turrax®, high pressure homogenisation) to build up the emulsion. A miniemulsion is a special case of emulsion. A mini-emulsion is obtained by shearing a mixture comprising two immiscible liquid phases, one surfactant and one co-surfactant. The shearing proceeds usually via ultrasonication of the mixture or with a high-pressure homogenizer, which are high-shearing processes (http://en.wikipedia.org/wiki/Miniemulsion, see also: Mini-emulsion Polymerisation, F. Joseph Schork et al., Adv Poly Sci (2005) 175: 129-255).

Document WO 2008/134734 A1 discloses a micelle having a contrast agent encapsulated therein, comprising a multiblock copolymer which comprises a polymeric hydrophilic block, optionally (amino acid block) that is optionally crosslinkable or crosslinked and another poly(amino acid) block, characterised in that said micelle has an inner core, optionally a crosslinkable or crosslinked outer core, and a hydrophilic shell. The formation of the micelles is based on crosslinked multiblock polymers. The disclosure does not provide a method, which enables a person skilled in the art to determine the number of nanoparticles per micelle.

The WO 2008/137733 A2 describes micellar structures, methods of making micellar structures, methods of imaging (e.g., imaging cancer and diseases and their related biological systems (e.g., proteins, antibodies, and the like associated with the cancer or disease)), methods of delivering therapeutic agents and/or biological compounds, and the like. The nanoparticles are encapsulated in amphiphilic block-copolymers without any information whether the number of nanoparticles per micellar structure can be adjusted.

The US 2004/033345A1 discloses a water-soluble complex comprising an inner core of a metal or semi-conductor nanoparticle, wherein the nanoparticle is coated with a hydrophobic ligand, which is encapsulated in a micelle. In an aqueous medium, the micelle comprises a hydrophilic shell and a hydrophobic core, the hydrophilic shell comprising a plurality of hydrophilic moieties, the hydrophobic core comprising a plurality of hydrophobic moieties, each hydrophobic moiety comprising at least one chain, each chain comprising a minimum of 8 atoms, wherein the total number of atoms in all chains for each moiety comprises at least 24 atoms. The micelle has a minimum average diameter of approximately 5 nm and a maximum average diameter of approximately 45 nm. The disclosure refers to a crosslinked static copolymer surrounding the nanoparticle, wherein it is not possible to encapsulate a certain number of nanoparticles.

Landfester et al. describes the encapsulation of iron oxide nanoparticles by a mini-emulsion polymerisation technique (J. Phys.: Condens. Matter, 2003, 15, 1345-1361). Here a high shear force by ultrasonication is used to disperse the nanoparticles in water. As surfactants next to sodium lauryl sulphate also polysorbate-80, lecithin and the sodium salt of cholic acid were used.

Paquet et al. describe the encapsulation of iron oxide nanoparticles by a mini-emulsion polymerisation technique (Langmuir, 2010, 26 (8), 5388-5396). Therein ultrasonication is used to disperse the nanoparticles in water.

However, the application of high shear forces is often disadvantageously to sensitive colloidal systems and severely limits the applicability. For instance, high shear forces will deteriorate the performance of nanoparticles like quantum dots e.g. fluorescence and quantum yield.

Ultrasonication is also not feasible if e.g. the molecules present in the emulsion have sensitive functionalities, delicate tertiary structures or labile chemical bonds, as e.g. common in proteins or polymers. Denaturation of proteins by ultrasonication is well known to the artisan. Deterioration of polymers by ultrasonication e.g. is well documented, e.g. Basedow et al. Die Makromolekulare Chemie, 1975, 176, 745-757.

Another drawback is the frequently limited solubility of the nanoparticles in the hydrophobic phase formed by the monomer. In order to completely dissolve a given amount of nanoparticles, a disproportionately high amount of monomer might be required. To overcome this issue co-solvents have to be employed, which need to be removed in later stages of the process. To this end, usually elevated temperatures are required (e.g. heating to 90 °C for 2 h was applied by Paquet et al. (Langmuir, 2010, 26 (8), 5388-5396), which again are often detrimental to sensitive components of the emulsion.

A further shortcoming of miniemulsion polymerisation techniques applied to the encapsulation of inorganic nanoparticles is that cluster size is difficult to control. Especially the generation of small clusters, particularly the encapsulation of single nanoparticles is severely restricted.

### Summary of invention

Surprisingly it was found that micro emulsion polymerisation techniques can be used for the encapsulation of nanoparticles. In a first aspect the present disclosure teaches a micellular combination that allows the stable dispersion of nanoparticles into aqueous environment. The micellular combination comprises at least one nanoparticle in a core of the micellular combination. A plurality of surfactants is co-assembled with a plurality of hydrophobic ligands on the surface of the nanoparticle in such a way that the hydrophilic part of the surfactant forms a hydrophilic shell around the core of the micellular combination. The hydrophilic shell ensures the dispersability in aqueous environments. A plurality of polymeric chains, which are comprised of at least one sort of polymerisable monomer, and which are optionally covalently interconnected comprise a matrix to which the pluralities of hydrophobic ligands and surfactants are optionally covalently attached, thus forming a controllable stable hydrophobic shell enclosing the nanoparticle in the core of the micellular combination.

In a further aspect the present invention also teaches a method for the manufacture of the disclosed micellular combination.

It is an advantage of the disclosed method that no energy has to be applied to the system for building up the emulsion e.g. by shearing (Ultra-Turrax®) or ultrasonication. This enables the technique to be applied to components witch can be easily damaged by such a treatment, like quantum dots, molecules with a labile chemical structure or polymers.

A further advantage of the disclosed method is that the thickness of the polymer shell can be adjusted by the amount of the monomer and/or an extension or reduction of the reaction time.

Polymerisable monomers, and optionally polymerisable surfactants and ligands produce a system with high stability against aggressive chemicals, biological fluids, e.g. whole blood, serum or plasma, aqueous solutions with high ionic strength, buffers, and micelle disassembly under high dilution conditions.

The number of inorganic nanoparticles incorporated into the micellular combination can be tuned via the nanoparticle to surfactant ratio. The technique allows encapsulating single nanoparticles or cluster of nanoparticles.

It is well known from the literature that a clustering of SPIONs into clusters containing a multitude of SPIONs can be advantageous for the modification of the magnetic properties of e.g. contrast agents for diagnostic imaging using magnetic resonance techniques or magnetic particle imaging

The invention discloses a method for the encapsulation of inorganic nanoparticles, bearing optionally polymerisable ligands on the surface. Said nanoparticles, which may be of one or different kinds, are co-dissolved with surfactants 20, which may be of one or different kinds in a water miscible organic solvent or mixtures thereof. Said surfactant 20 is comprised of an optionally polymerisable hydrophobic moiety 40 and a hydrophilic moiety 30. Optionally the organic solution obtained by mixing inorganic nanoparticle and surfactant may comprise additionally a hydrophobic polymerisation initiator. Said organic solution is transferred into an aqueous solution, in which a micellular combination is formed spontaneously, wherein a plurality of said surfactants 20 encloses at least one inorganic nanoparticle per micellular combination. A polymerisable monomer and optionally a hydrophilic polymerisation initiator are mixed and a polymerisation reaction is performed, thus generating the micellular combination 90.

In one aspect, the method discloses a controlled volume flow of the polymeris able monomer to the reaction site of the polymerisation reaction inside the hydrophobic region of the micellular combination 90.

In a further embodiment of the invention said method can be executed as a batch process.

In a further embodiment of the invention said method can be executed as a semi-batch process, e.g. feeding a monomer solution into the reaction mixture.

In a further embodiment of the invention said method can be executed as a continuous flow process.

The present disclosure also teaches the use of the micellar combination, for example as a contrast agent in radiological diagnosis imaging, in medical imaging and in therapeutic applications. Consequently, the disclosed micellular combinations are intended for use in in vitro, in vivo and/or in situ imaging or staining of synthetic or biological molecules or cellular structures like membranes, peptides or other cellular organelles.

### Brief description of the figures

The disclosure will be illustrated by figures and examples without being limited to the described embodiments.
Figure 1a shows a schematic overview of the micellular combination 90
Figure 1b shows a schematic overview of the micellular combination 91
Figure 1c shows a schematic overview of the micellular combination 92
Figure 2 shows a schematic overview of a reaction according to an aspect of the present invention, in which a hydrophobic moiety of a surfactant 20 and the ligands of the nanoparticle form a micellular combination 90.
Figure 3 shows a) cluster size as a function of amount of nanoparticles to amount of surfer ligands, b) DLS measurement (volume) as a function of diameter of the cluster according to example 2.
Figure 4 shows TEM images of the products described in figure 7, showing uniform micellular combinations 90 according to example 2.
Figure 5 shows a transmission electron microscopy image of a micellular combination 90 comprising iron oxide nanoparticles according to example 3.
Figure 6 DLS measurement (intensity) as a function of diameter of the micellular combination 90, according to example 3.
Figure 7 shows transmission electron microscopy images of a) left and b): iron oxide nanoparticles and a) right: gold nanoparticles according to example 4. a)
Figure 8 shows a schematic representation (DLS measurement (intensity and volume)) of the polymer shell growth during formation of the micellular combination 90 according to the present disclosure, by adding different amounts of monomer according to example 5.
Figure 9 shows a schematic representation (DLS measurement (intensity) of the polymer shell growth during formation of the micellular combination 90 according to the present disclosure, by prolonging the reaction time according to example 6.
Figure 10 shows a schematic representation of the polymer shell growth during formation of the micellular combination 90 according to the present disclosure, by prolonging the reaction time according to example 6. Figure 10 a: transmission electron microscopy image take of the sample after 5 min of polymerisation. Figure 10 b: transmission electron microscopy image take of the sample after 90 min of polymerisation.
Figure 11 shows a transmission electron microscopy image of a micellular combination 90 comprising iron oxide nanoparticles according to example 7.
Figure 12 DLS measurement (intensity) as a function of diameter of the micellular combination 90, according to example 7.
Figure 13 shows a transmission electron microscopy image of a micellular combination 90 comprising iron oxide nanoparticles according to example 8.
Figure 14 DLS measurement (intensity) as a function of diameter of the micellular combination 90, according to example 8.
Figure 15 shows transmission electron microscopy images of a micellular combination 90 comprising iron oxide nanoparticles according to example 9.
Figure 16 DLS measurement (intensity) as a function of diameter of the micellular combination 90, according to example 9.
Figure 17 shows transmission electron microscopy images of a micellular combination 90 comprising iron oxide nanoparticles according to example 10.
Figure 18 DLS measurement (intensity) as a function of diameter of the micellular combination 90, according to example 10.
Figure 19 shows a transmission electron microscopy image of a micellular combination 90 comprising iron oxide nanoparticles according to example 11.
Figure 20 DLS measurement (intensity) as a function of diameter of the micellular combination 90, according to example 11.
Figure 21 shows a transmission electron microscopy image of a micellular combination 90 comprising iron oxide nanoparticles according to example 12.
Figure 22 DLS measurement (intensity) as a function of diameter of the micellular combination 90, according to example 12.
Figure 23 shows a transmission electron microscopy image of a micellular combination 90 comprising InP nanoparticles according to example 13.
Figure 24 DLS measurement (intensity) as a function of diameter of the micellular combination 90, according to example 13.
Figure 25 shows transmission electron microscopy images of a micellular combination 90 comprising CdSe/CdS/ZnS *core*/*shell*/*shell* quantum dot nanoparticles according to example 14.
Figure 26 DLS measurement (intensity) as a function of diameter of the micellular combination 90, according to example 14.
Figure 27 shows the proportionality of fluorescence and concentration of the micellular combination 90, according to example 14. The proportionality shows stability of the micellular combination 90.
Figure 28 shows a transmission electron microscopy image of a micellular combination 90 comprising iron oxide and gold nanoparticles according to example 15.
Figure 29 DLS measurement (intensity) as a function of diameter of the micellular combination 90, according to example 15.
Figure 30 shows a transmission electron microscopy image of a micellular combination 90 comprising fluorinated polymerisable groups according to example 16. The Fe- and F-ratio was determined by EDX.
Figure 31 shows transmission electron microscopy images of a micellular combination 90 comprising CdSe/CdS/ZnS *core*/*shell*/*shell* quantum dot nanoparticles and sqalene as filler according to example 17.
Figure 32 DLS measurement (intensity) as a function of diameter of the micellular combination 90, according to example 17.

### Detailed description of the invention

For a complete understanding of the present disclosure and the advantages thereof, reference is made to the following detailed description taken in conjunction with the accompanying figures.

It should be appreciated that the various aspects of the present disclosure as disclosed herein are merely illustrative of specific ways to make and use the invention and do not therefore limit the scope of disclosure when taken into consideration with the appended claims, the detailed description and the accompanying figures.

It should be realised that features from one aspect of the disclosure will be apparent to those skilled in the art from a consideration of the description or examples of the disclosure herein and such features can be combined with features from other aspects of the disclosure.

A "functional group" should be understood in the context of the present disclosure as a chemical entity e.g. a group of covalently arranged atoms, which interact in a predictable way for a specific purpose. In one aspect of the invention said purpose can be the attachment of an organic molecule to the surface of an inorganic nanoparticle.

In a further aspect of the invention the purpose can be the attachment of a linker to the polymer shell of the micellular combination.

In a further aspect of the invention the purpose can be the attachment of an affinity molecule, cabable of interacting with biological entities

In a further aspect of the invention the purpose can be the transformation into another functional group. Halogen, if not specified in the text includes fluoro, chloro, bromo and iodo.

The term "center" in the context of the present disclosure designates the inorganic nanoparticle 10, which is enclosed by any part of the micellular combination 90, not necessarily corresponding to the geometrical center of the micellular combination 90.

For starting a radical polymerization, many different techniques (homolysis, thermal initiation, induced decomposition, electron transfer, photoinitiation, ultrasound initiation, radiation initiation, etc.) are known in literature (M. Krzysztof, Handbook of radical polymerization, Wiley-Interscience, 2002). In the present invention the initiation of the polymerisation should not be understood to be limited concerning the initiation technique.

The terms batch, semi-batch, and continuous flow process should be understood as common in the literature (M. Krzysztof, Handbook of radical polymerization, Wiley-Interscience, 2002, p.339 ff.). In one aspect of the invention the process (batch, semi-batch, and continuous flow) should be only referred to the emulsion polymerisation. In a further aspect of the invention the transfer of the organic solution into the aqueous solution should also be included into the whole process. If the transfer of the organic solution into the aqueous solution is included, "semi batch" is defined as feeding of a monomer and /or initiator or a solution thereof to the reaction vessel.

"Transfer into" in the context of creating a mixture of an organic solution and an aqueous solution within the spirit of the present disclosure should be understood that the transfer may be carried out by different technical means, e.g. manually or mechanically, using e.g. dropping funnels, syringes, or pumps e.g. syringe pumps, jets, and the like. Furthermore, it should be understood that said transfer can be achieved at various feeding rates, e.g. dropwise or rapid.

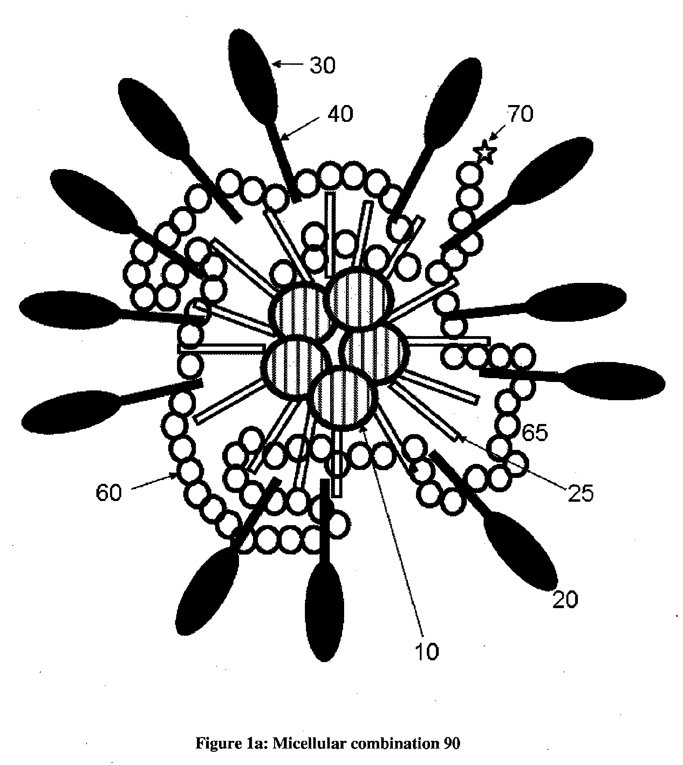

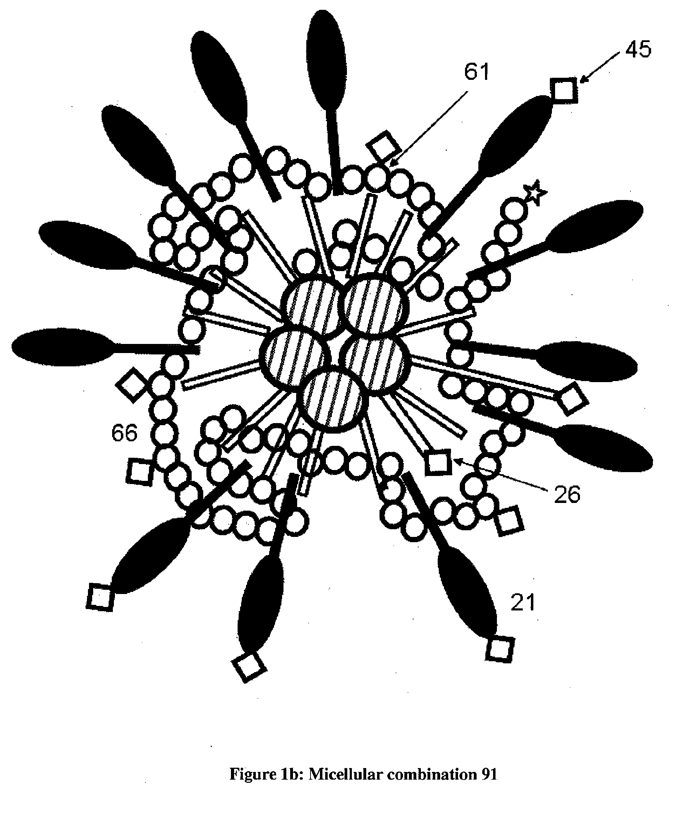

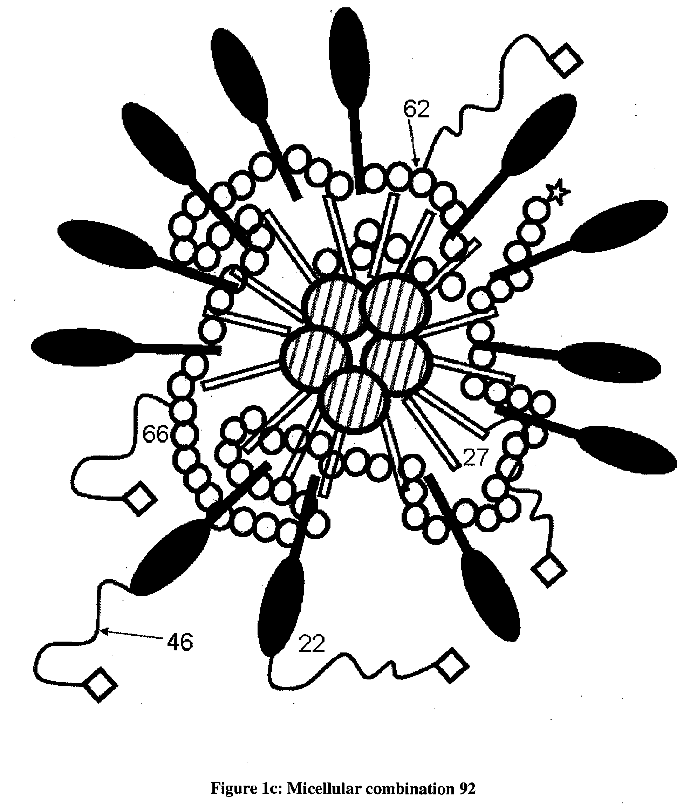

Figure 1 shows a schematic overview of the micellular combination 90.

The present disclosure provides a micellular combination 90 and in particular a water stable micellular combination 90. Referring to Figure 1 the micellular combination 90 comprises an inorganic nanoparticle 10, bearing a hydrophobic ligand 25 on the surface , a surfactant 20 which in some embodiments is a polymerisable surfactant termed surfmer and a monomer which polymerises inside the hydrophobic region of the micellular combination 90. Addition of functional monomers or surfactants during the process of manufactur allows for the adjustment of the surface properties of the micellular combination 90.

The inorganic nanoparticle 10 can be any of a metal nanoparticle (e.g. Au, Ag, Pt, Pd, Co, Ni, or alloys like NiₓPt_{y}, CoₓPtyₓ, FeₓPt_{y}, NiₓPd_{y}, CoₓPd_{y}, FeₓPd_{y}), metal oxide nanoparticle (e.g. Iron oxide, TiO₂), semiconductor nanoparticles or rare earth doped nanoparticles (e.g. YVO₃:La LaPO₄:Ce. Non-limiting examples of the semiconductor quantum dot materials include, CdSe, CdS, ZnSe, ZnS, CuS, CuSe (II-VI materials), PbS, PbSe, PbTe (IV-VI materials), InN, InP, InAs (III-V materials) and InSe, GaSe (III-VI materials). The semiconductor nanoparticle 10 can also be a core/shell (e.g. CdSe/CdS, CdSe/ ZnS)) or core/shell/shell nanoparticles (e.g. CdSe/CdS/ZnS) In one aspect of the present invention the semiconductor nanoparticles are made of mixed selenides like CuₓIn_{y}Ga_{z}Se (CIGS), CuₓIn_{y}Se, CuₓGa_{y}Se, InₓGa_{y}Se.

Optionally, said nanoparticles may comprise radioactive isotopes.

The hydrophobic ligand 25 is a molecule with a group with an affinity to the inorganic nanoparticle surface and a hydrophobic moiety. The hydrophobic moiety can be branched (TOP) or linear (e.g. oleylamine). The ligand can be trioctylphosphine (TOP) or trioctylphosphine oxide (TOPO) or saturated and (termihally)-unsaturated alkyl chains with terminal adhesion groups like thiols, amines, carboxylic acids, phosphines, phosphine oxides, silanes. Some of them can already be present during nanoparticle synthesis. In some aspects of the present disclosure the alkyl chains have bi-or multi-dentate affinity groups like for example diethylenetriamine, a dithiocarbamate, affinity group or a dihydrolipoic acid terminus. In some aspects of the present disclosure the hydrophobic ligand 25 can also be a polymer with an affinity group like for example polyisoprene-diethylenetriamine (PI-DETA). The polymer can be either fully saturated or in some embodiments of the present disclosure it contains polymerisable units like double or triple bonds.

The general structural properties of the surfactant 20 are that it is an amphiphilic molecule that is able to lower the surface tension of a biphasic system. The surfactant 20 encapsulating the hydrophobic core of the micellular combination 90 can either be ionic or non-ionic in its hydrophilic region. Ionic groups can also be zwitter ions or betaine structures, like carboxy betaine, phospho betaine, e.g. phophatidylcholines, phosphono betaine or sulfo-betain structures. When the surfactant 20 has polymerisable units in its hydrophobic region, which can be copolymerised in a chain reaction, it is termed surfmer. Surfmer ligands are known in art. The term "surfmer" derives from the word surfactant and monomer. The surfactant or surfmer respectively can either be a low molecular weight molecule with a defined structure or an amphiphilic di- or multi-block copolymer.

An example for a non-ionic surfactant with a polymerisable carbon-carbon double bond is the commercially available polysorbate-80 surfmer (Tween® 80 - Trademark is registered to ICI). The polysorbate-80 surfmer is a non-ionic surfactant and a monomer that is derived from polyethoxylated sorbitan and oleic acid. The hydrophilic moiety 30 of the polysorbate-80 comprises polyethers. The hydrophobic moiety 40 of the surfmer ligand contains a carbon-carbon double bond and the hydrophobic moiety 40 is an ester of oleic acid. In certain embodiments the surfactant 20 can be an amphiphilic di or multi-block copolymer comprising polymerisable units like double or triple bonds in its hydrophobic section. An example for such a polymer is b-polyisopren-b-polyethylene oxide. The layer of surfactant 20 around the hydrophobic core of the micellular combination 90 can also contain a mixture of two or more different surfactants 20.

In certain embodiments of the present disclosure a layer of emulsifiers not comprising any polymerisable units in their hydrophobic moiety can also encapsulate micellular combination 90. Examples for such emulsifiers are sodiumdodecylsulfate (SDS) or sodium dodecyl benzene sulfonate (SDBS).

To introduce functional groups on the surface of the micellular combination 90 it is possible to introduce other functional monomers that show detergent-like behaviour (e.g. Ralu®mer SPV from Raschig GmbH). Functional monomers, that do not stabilize the particle dispersion in water alone can be used in mixtures together with SDBS for example.

A monomer that can polymerise inside the hydrophobic core of the micellular combination is used. The monomer or the monomer mixture used to polymerise the existing polymerisable units in the hydrophobic region of the micellular combination 90 is poorly soluble in water. Monomers that can be used include acrylates (e.g. ethylacrylate), methacrylates (e.g. methylmethacrylate, allyl methacrylate, acrylic acid, methacrylic acid (MA), methyl methacrylate (MMA), styrene (ST) or vinyl benzene (VB), ethyl acrylate (EA), butyl acrylate (BA), 2-ethylhexyl acrylate (EHA), dimethylaminoethyl methacrylate (DMAEMA), tert-butylaminoethyl methacrylate (TBAEMA), hydroxyethyl methacrylate (HEMA), and hydroxypropyl methacrylate (HPMA), acetylacetoxyethylmethacrylate) olefins (e.g. isoprene) or vinyl benzene and its derivatives. The derivatives of vinyl benzene and/or divinyl benzene can include fluorinated vinyl benzene and/or fluorinated divinyl benzene. The derivatives of vinyl benzene and/or divinyl benzene can be a mixture of -ortho and -para isomers of vinyl benzene and/or divinyl benzene. Functional monomers that do not possess an amphiphilic character so that they can be applied together with a co-surfactant can be mixed with the monomer so that it is incorporated in a statistical manner in the micellular combination 90.

The polymerisation initiator 70 is a molecule that forms an active species through thermal, photochemical or redox stimulus wich can be transferred to a monomer that starts the polymer chain reaction. In the context of this invention the initiator 70 is used to start the polymerisation of the monomer inside the hydrophobic core of the micellular combination 90. An example for a molecule that is initiated thermally is dibenzoylperoxide and a photochemical initiator is (e.g. DAROCUR® 1173). Initiator 70 can be used as a single species or as a mixture of initiator species. The polymerisation initiator 70 can be a water-soluble polymerisation initiator 70 or an hydrophobic polymerisation initiator 70. The water-soluble polymerisation initiator 70 can be 2, 2'-Azobis[2-(2-imidazolin-2-yl) propane] dihydrochloride (VA-044) or sodium peroxodisulphate (NaPs). The hydrophobic polymerisation initiator 70 can be azobisisobutyronitrile (AIBN). The polymerisation initiator 70 can be a water-soluble polymerisation initiator 70 or an hydrophobic polymerisation initiator 70 depending on the solvents used in the reaction.

In some cases it can be desirable to make the core of the micellular combination 90 more hydrophobic. This can be achieved by addition of a hydrophobic filler molecule. Such filler molecules can be long alkyl chains containing polymerisable units like double or triple bonds like e.g. squalene. They also can be seen as a hydrophobic co-monomer. In the case of squalene the function can be twofold. In addition to its hydrophobic character it is also known to act as an antioxidant. This can be advantageous when the hydrophobic core comprises semiconductor quantum dots because an antioxidant can quench singlet oxygen, which reduces the fluorescence intensity of semiconductor quantum dots.

The micellular combination 90 comprises a hydrophobic core in the centre of the micellular combination 90. The core of the micellular combination 90 comprises at least one inorganic nanoparticle 10.

Surrounding each inorganic nanoparticle 10 of the micellular combination 90 is a shell of surface attached hydrophobic ligands 25 (Figure 1) which are attached to the inorganic nanoparticle surface through adhesion groups. In certain embodiments the hydrophobic ligand 25 can contain polymerisable functional units and /or polymerisable double or triple bonds. Typically after their synthesis the inorganic nanoparticles are hydrophobic on their outer surface and have trioctyl phosphin (TOP), trioctylphosphine oxide (TOPO) and monoalkyl surfactants (e.g. hexadecylamine, oleic acid) on their surface. These original synthesis ligands can be exchanged by reacting the synthesised nanoparticles in an excess solution of a new ligand. This so called ligand exchange is a standard procedure for nanoparticle dispersions. Through such a ligand exchange the hydrophobic ligand 25 containing polymerisable units can be introduced to the surface of the inorganic nanoparticle 10. In one aspect of the present disclosure the synthesis ligands are not replaced by a polymerisable hydrophobic ligand 25 but the nanoparticles 10 are used without post-synthetic treatment. A plurality of hydrophobic ligand 25 species can be present on the nanoparticle surface at the same time.

The inorganic nanoparticles 10 with the attached hydrophobic ligand 25 form the hydrophobic core of the micellular combination 90. The number of inorganic nanoparticles 10 in the core is not limited. In some aspects of the present disclosure the preparation of clustered inorganic nanoparticles inside one micellular combination 90 can result in altered physicochemical properties of the system. For example it is well known that clusters of super paramagnetic iron oxide nanoparticles retain their super paramagnetic properties while showing very high relaxivities r2 which are usually found for SPIONs of much higher diameter. The high relaxivity renders the SPION clusters useful for magnetic resonance imaging. The number of nanoparticle species inside the cluster is not limited to one. It can also contain a mixture of two or more different kinds of nanoparticles.

The surfactants 20 comprising a hydrophilic moiety 30 and a hydrophobic moiety 40 are assembled around the core of the micellular combination 90 with the hydrophobic moiety 40 pointing away from the hydrophilic environment. By using different surfactant species it is possible to introduce different functionalities on the hydrophilic surface of the micellular combination 90.

In some embodiments of the present disclosure an established surfactant without polymerisable units, like SDS or SDBS is used to surround the hydrophobic core by forming a self-assemble layer. This is sufficient to stabilize the nanoparticles 10 in aqueous environment and allow for polymerisation inside the hydrophobic region of the micellular combination 90.

In one embodiment of the present disclosure a surfactant 20 is used that contains polymerisable units in its hydrophobic moiety 40 and which is then also named a "surfmer"

In an aspect of the present invention a polymensable monomer is used to form a polymeric shell inside the hydrophobic region of the micellular combination 90, which encloses the embedded inorganic nanoparticles in a polymer matrix inside the hydrophobic core of the micellular combination 90 and protects them from the environment.

In some embodiments the in-situ formed polymeric shell encapsulates the inorganic nanoparticles 10 in the hydrophobic core of the micellular combination without copolymerising the hydrophobic ligand 25 and the hydrophobic moiety 40 of the surfactant shell. The resulting polymer encloses the inorganic nanoparticles 10 in the hydrophobic core purely by hydrophobic interactions.

In one aspect of the present invention the polymerisable monomer copolymerises with the polymerisable units of the hydrophobic ligand 25 to form a polymeric shell but does not covalently polymerise the hydrophobic moiety 40 of the surfactant ligand 20. After the synthesis of the micellular combination 90, which was constructed in this way, the surfactant 20 can be removed, e.g. *via* dialysis. Thus, an inorganic-polymer hybrid latex particle with a hydrophobic outer surface is formed.

In a further aspect of the present invention a number polymerisable monomers 60 copolymerises with the polymerisable units in the hydrophobic moiety 40 of the surfactants 20 to form a polymeric shell, which encapsulates the inorganic nanoparticle or a multitude thereof in the micellular combination 90.

In one embodiment of the present invention the polymerisable units in the hydrophobic ligand 25 and the polymerisable units in the hydrophobic moiety 40 of the surfactant 20 are both copolymerised with the monomer 60 to substantially polymerise all components in the polymeric shell. This creates a hybrid latex particle where inorganic nanoparticles are irreversibly embedded in a polymer matrix, which makes the particles insensitive to high dilution conditions. Below the critical micelle concentration a micellular aggregate will disassemble into its constituting amphiphiles if it is not polymerised..

In some embodiments of the present invention optionally a hydrophobic filler is copolymerised to increase hydrophobicity of the micelle core. Said filler may comprise of squalene, which furthermore is known to act as an antioxidant to quench singlet oxygen, thus stabilising components of the micellular combination, which are prone to oxidation.

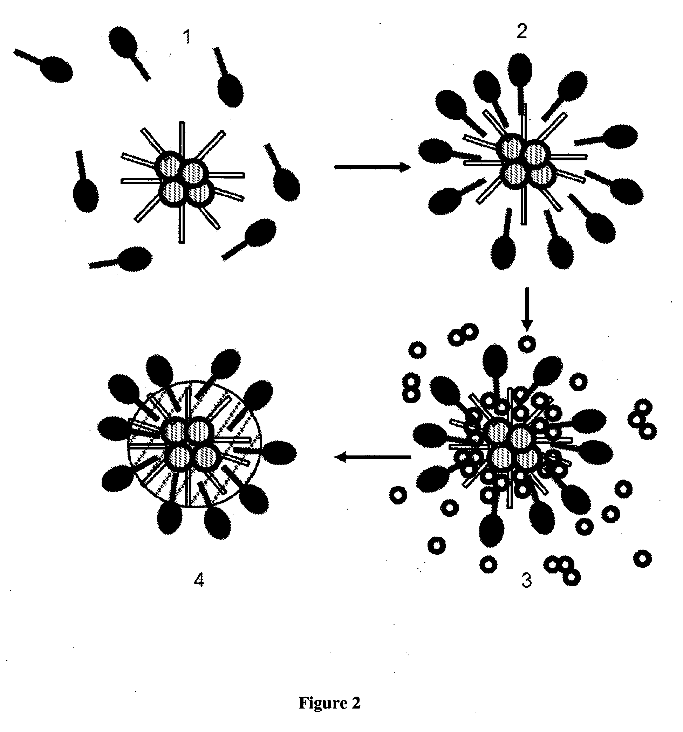

Figure 2 illustrates the method to manufacture the micellular combination 90. In step 1 of figure 2 (top left ), the inorganic nanoparticle 10 is mixed with the surfactant 20 in a water miscible organic solvent or a water miscible mixture of organic solvents. For example the organic solvent is tetrahydrofuran or acetonitrile or mixtures thereof. In the case where a hydrophobic initiator is used it is also added to the organic solution (not shown in the scheme). Functional monomers that are surface active in some aspects of the present invention are also mixed in the organic solution with the surfactants 20 and the nanoparticles 10.

In step 2 of figure 2 the solution of surfactant 20 and the nanoparticles are transferred to water and the surfactants 20 surround the hydrophobic core enclosing the nanoparticles 10. In particular the hydrophobic moiety 40 surrounds the hydrophobic core of the micellular combination. The surfactant 20 forms a self-assembled layer around the hydrophobic core comprising inorganic nanoparticles 10 as shown in figure 2, step 2. Through the variation of the mixing ratio of nanoparticles 10 and surfactant 20 the number of nanoparticles 10 per cluster can be adjusted. In the spirit of the invention at least a single nanoparticle 10 is present in one micellular combination 90. If the micellular combination contains a multitude of nanoparticles, said nanoparticles may be of one or different kinds.

In step 3 of figure 2, a polymerisable monomer 60 is added to the aqueous phase. Albeit the aqueous solubility of the polymerisable monomer is low, a diffusion of solubilized monomer from the aqueous phase into the particle loaded micellular combination 90 takes place, thus increasing the thickness of the polymer shell during the polymersisation reaction.

In step 4 of figure 2, a water soluble polymerisation initiator 70 (not shown) is added, thus starting the emulsion polymerisation reaction. In the case of an aqueous initiator the reaction is initiated in water where oligomers grow. In the course of the reaction the oligomers become more and more hydrophobic and migrate into the micellular combination 90 where the polymerisation reaction continues. Due to the low solubility the monomer flow from the monomer emulsion to the micelles the reaction is dominated by diffusion controlled mass flow into the micellular combination. The thickness of the polymer shell can be controlled by adjusting the reaction time or through the amount of monomer added to the reaction mixture or combinations thereof.

In a further embodiment of the invention a hydrophobic initiator 70 is added to the water miscible organic solution prior to the transfer into water. After micelle formation the polymerisation reaction of the polymerisable monomer 60 is started directly inside the micellular combination 90.

In one aspect of the invention the hydrophobic moiety 40 of the surfactant 20 and/or the hydrophobic ligand 25 may bear polymerisable double or triple bonds, which are co-polymerised with the polymerisable monomer 60 to form a polymeric shell, thus providing a polymerised hybrid latex particle where the inorganic nanoparticle 10 is enclosed in a polymeric matrix.

In one aspect of the disclosure, surfactant 20 provides terminal or non-terminal functional groups in the hydrophilic moiety 30, which are suitable for the functionalization with affinity molecules capable of interacting with biological entities.

In a further embodiment of the disclosure each component, e.g. a plurality of surfactants, a plurality of hydrophobic ligands, a polymerisable monomer or any combinations thereof taking part in the polymerisation reaction of the micellular combination 90, may optionally bear orthogonal functional groups or affinity molecules capable of interacting with biological entities, optionally attached via an interjacent linker or any combination thereof, thus forming a micellular combination 91 or 92 (Figures 1a and 1b). Said affinity molecules may interact *in vivo, ex vivo* or *in vitro* with biological entities, like tissues, cells, viruses, receptors, membranes, antibodies, ion channels, nucleotide sequences, enzymes, lectins etc. Said affinity molecules can be small druglike molecules, peptides, proteins, e.g. antibodies or fragments thereof, mono-, oligo-, or polysaccharides, or nucleotide sequences, e.g. aptamers, which are not affected by the mild polymerisation reaction conditions. Said affinity molecules may be optionally protected by means of protecting groups, well known to the artisan, e.g. as referenced in "Protective Groups in Organic Synthesis" by Peter G. M. Wuts, Theodora W. Greene, Wiley & Sons, 2006. Said protective groups may optionally be removed after formation of the micellular combination 91 or 92. The micellular combination 90 optionally modified with affinity molecules capable of interacting with biological entities are useful as biomarkers, probes, therapeutics, or *in vivo, ex vivo* or *in vitro* diagnostic agents. For example, the components participating in the emulsion polymerisation process may bear functional groups orthogonal to the polymerisation conditions, e.g. these functional groups are not involved in the polymerisation reaction and remain stable. For instance, the polymerisable monomer may bear said functional groups. Said functional groups can be optionally transformed consecutively under reaction conditions different from the polymerisation reaction conditions. For example, the polymerisable monomer is comprised of 2,3,4,5,6-penta fluoro vinyl benzene, solely or in combination with other polymerisable monomers. It is known from the literature that the fluoro atom in the 4-position of 2,3,4,5,6-penta fluoro vinyl benzene is amenable to nucleophilic displacement under mild conditions, e.g. by thiols like thioglycosides [C. Remzi Becer et al., Macromolecules 2009, 42, 2387-2394] or nucleophilic amines and the like. For instance, if the functional group is an azido moieties, it can participate in 1,3-dipolar cycloaddition reactions, well known in the literature ("Click"-chemistry). In another example the polymerisable monomer is comprised of derivatives, e.g. esters of acrylic or methacrylic acids or the like, solely or in combination with other polymerisable monomers, in which the ester functionality may be transformed consecutively after polymerisation of the micellular combination 90. Said transformations may consist in the attachment of affinity molecules capable of interacting with biological entities, optionally via an interjacent linker. Said linkers may comprise of polyethers, e.g. polyethylene glycol, polypropylene glycol and the like.

Said affinity molecules capable of interacting with biological entities may be attached via common coupling procedures, well known in the art, e.g. using EDC NHS or Sulfo-NHS reagents or the like.

A further embodiment of the invention includes a process for the generation of a micellular combination 91 or 92, in which components, e.g. said surfactants, hydrophobic ligands, and polymerisable monomer bear optionally combinations of orthogonal functional groups, linkers, or affinity molecules capable of interacting with biological entities, thus allowing convenient modifications of the micellular combination 91 or 92 in a wide range of biological properties.

### Examples

Example 1. Encapsulation of nanoparticles by seed emulsion polymerisation.

The encapsulation of nanoparticles by seed emulsion polymerisation was carried out according to the following general conditions. Nanoparticles free of storage solvent were taken up in THF. Emulsifier was added and initiator for the radical polymerisation, for the case of a hydrophobic initiator, was added. If the taking up procedure of the nanoparticles should be speeded up the THF solution can be treated 5 min with an ultrasonic bath. The use of the ultrasonic bath is not required in any case. The THF solution was then injected into water. 1-pentanol and monomer was added. Finally initiator for the radical polymerisation, for the case of a hydrophilic initiator, was added and the solution was heated and stirred up to 72 hours at a temperature of between 15 °C to 100 °C. The isolation of the micellular combination, were the core is loaded with SPION, can be performed with magnetic columns.

In a variation on the encapsulation of nanoparticles by seed emulsion polymerisation (Example 1), the addition of the emulsifier and initiator for the radical polymerisation was carried out in the nanoparticle storage solvent chloroform. The nanoparticle storage solvent chloroform was then removed under a stream of nitrogen gas and THF was added to the resulting residue.

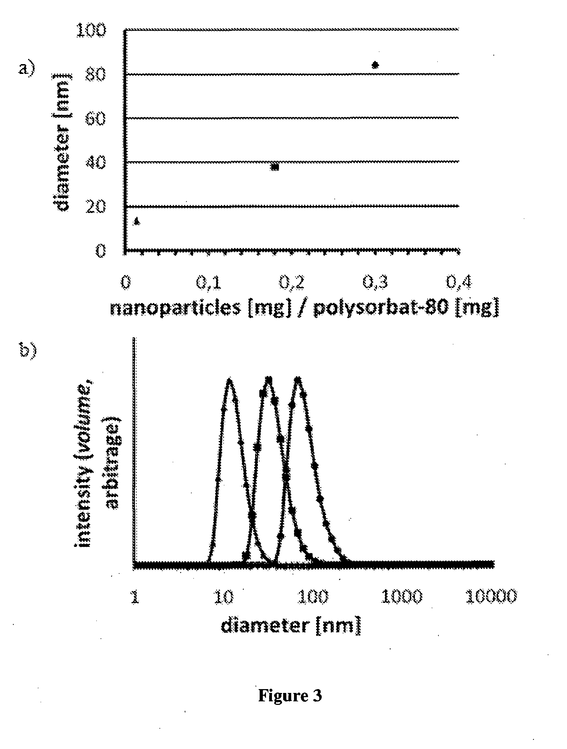

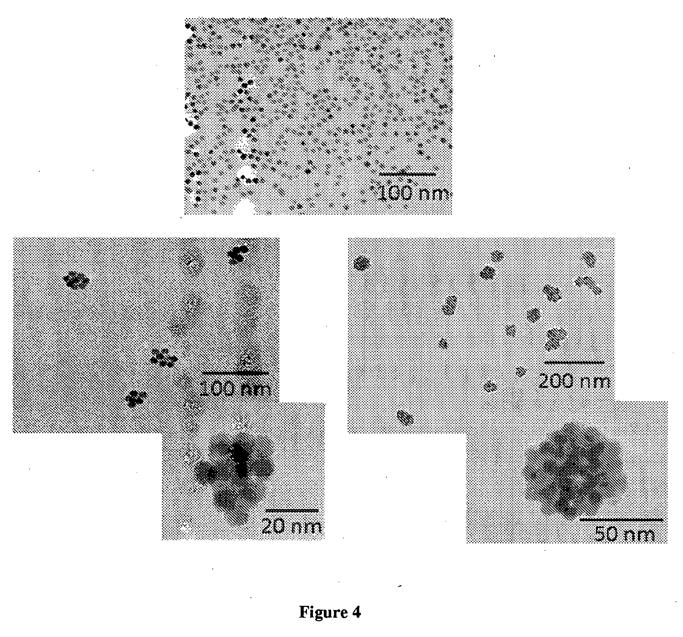

Example 2. Example 1 as above was carried out with the following specific conditions. The results are shown in figure 3 and 4.

Example 2a (▲) - 4.6 nmol (5.2 mg) of iron oxide nanoparticles (oleic acid coated), 404 mg Polysorbate-80, 2 mL of THF, 50 µL 1-pentanol, 0 µL vinyl benzene, 0 µL divinyl benzene, 20 mL water, 2 mg AIBN, were stirred for 15 hours at 70 °C. Example 2b (■): 0.46 nmol (0.52 mg) of iron oxide nanoparticles (oleic acid coated), 0.3 mg polysorbate-80, 0.3 mL of THF, 2 µL 1-pentanol, 0 µL vinyl benzene, 0 µL divinyl benzene, 3 mL water, 1 mg AIBN, were stirred for 15 hours at 75 °C. Example 2c (●): 2.3 nmol (2.6 mg) of iron oxide nanoparticles (oleic acid coated), 0.9 mg polysorbate-80, 1.5 mL THF, 0 µL 1-pentanol, 0.42 µL vinyl benzene, 0.08 µL divinyl benzene, 15 mL water, 1 mg VA-044, were stirred for 15 hours at 75 °C.

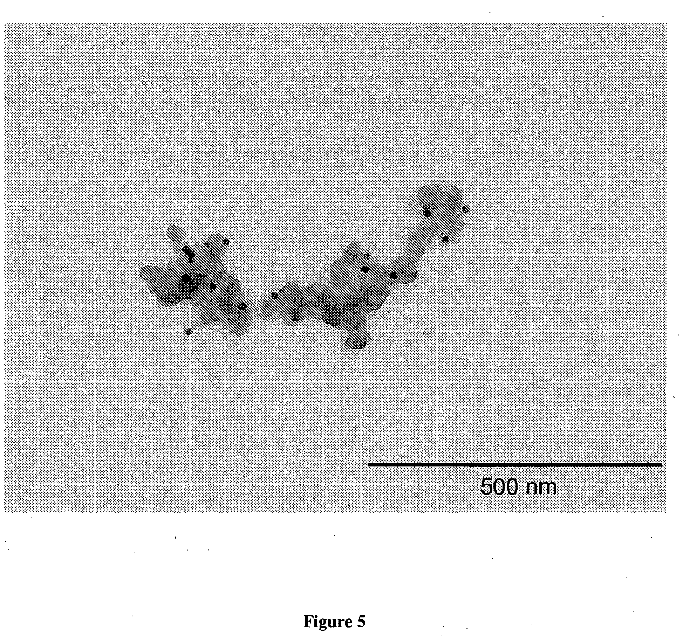

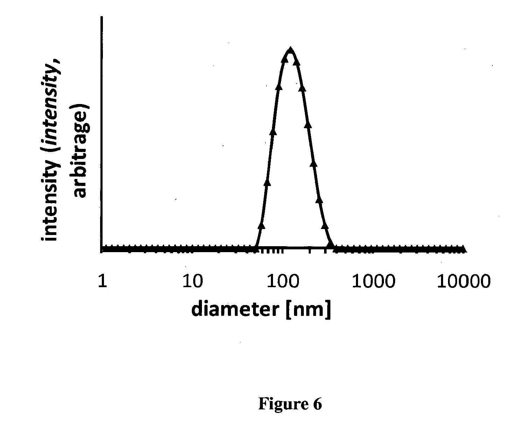

Example 3. Example 1 as above was carried out with the following specific conditions. The results are shown in figure 5 and 6.

1.1 nmol of iron oxide nanoparticles (oleic acid coated), 10 mg SDBS, 3 mL of THF, 30 µL 1-pentanol, 15 µL vinyl benzene, 15 µL divinyl benzene, 20 mL water, 1 mg VA044, were stirred for 15 hours at 50 °C.

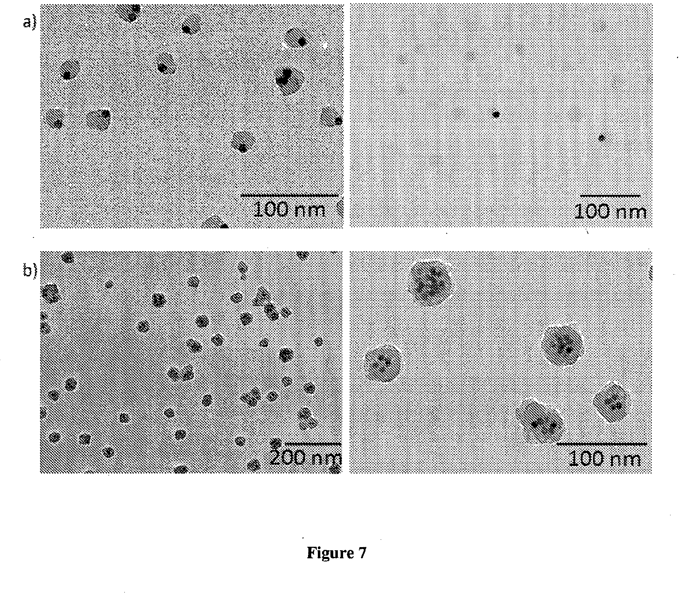

Example 4. Example 1 as above was carried out with the following specific conditions. The results are shown in figure 7.

Figure 7: a) left: 1.8 nmol iron oxide nanoparticles (oleic acid coated), 3.0 mg polysorbate-80, 2 mL of THF, 5 µL 1-pentanol, 25 µL vinyl benzene, 10 µL divinyl benzene, 20 mL water, 2 mg VA-044, were stirred for 15 hours at 44 °C. Figure 7: a) right: 0.33 nmol gold nanoparticles (oleylamine coated), 1.1 mg polysorbate-80, 2 mL of THF, 5 µL 1-pentanol, 25 µL vinyl benzene, 10 µL divinyl benzene, 20 mL water, 2 mg VA-044, stirred for 15 hours at 44 °C, and then for 2 hours at 65 °C. Figure 7 b) 0.46 nmol iron oxide nanoparticles (oleic acid coated), 2.0 mg of polysorbate-80, 2 mL of THF, 5 µL 1-pentanol, 25 µL vinyl benzene, 10 µL divinyl benzene, 20 mL water , 2 mg VA-044, was stirred for 15 hours at 44 °C and then 2 hours at 75 °C.

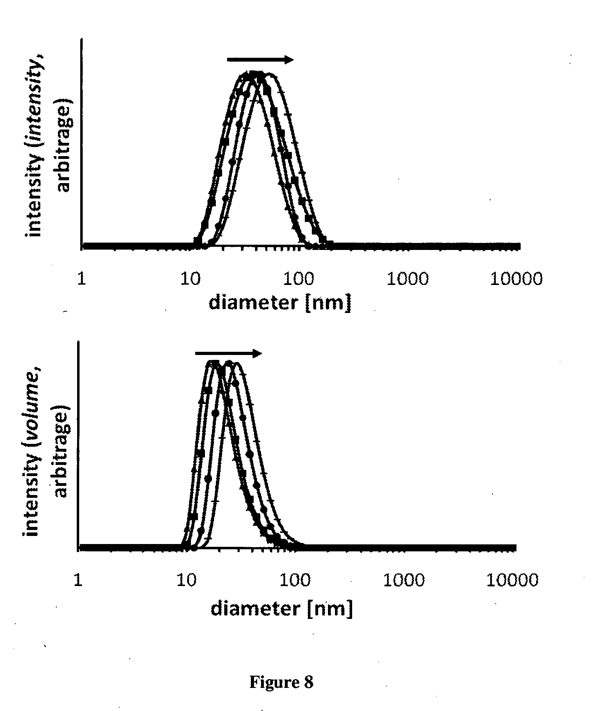

Example 5. Example 1 as above was carried out with the following specific conditions. The results are shown in figure 8.

0.46 nmol iron oxide nanoparticles (oleic acid coated), 3.0 mg polysorbate-80, 0.3 mL of THF, 0 µL 1-pentanol, 3 mL water, 1 mg AIBN, were stirred for 5 hours at 75 °C. ▲: 0.17 µL vinyl benzene, 0.03 µL divinyl benzene. ■: 1.0 µL vinyl benzene, 0.20 µL divinyl benzene. Circle line ●: 2.5 µL vinyl benzene, 0.50 µL divinyl benzene. Dashed line -: 5.0 µL vinyl benzene, 1.0 µL divinyl benzene.

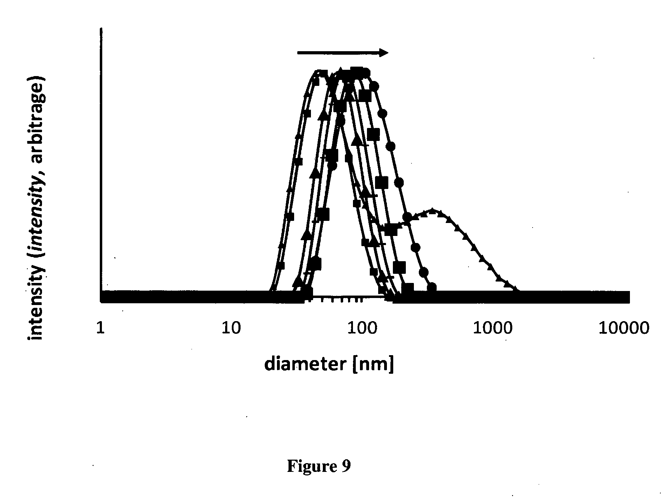

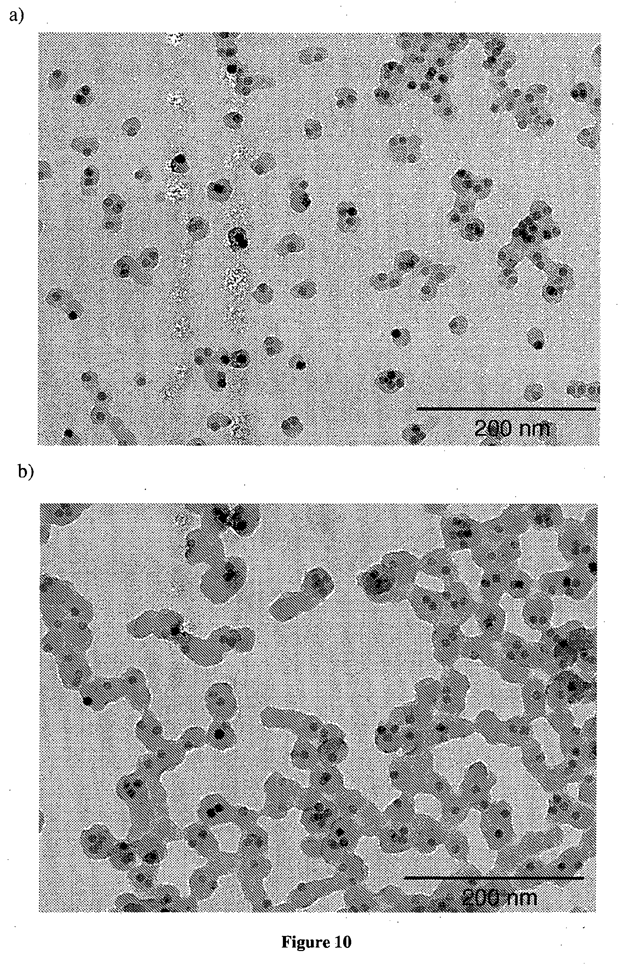

Example 6. Example 1 as above was carried out with the following specific conditions. The results are shown in figure 9 and 10

23 nmol iron oxide nanoparticles (oleic acid coated), 44 mg PI-PEO (M = 3800 g/mol), 2.5 mL of THF, 30 µL 1-pentanol, 20 mL water, 169 µL µL vinyl benzene, 169 µL divinyl benzene 1 mg VA-044, were stirred for 90 min at 50 °C. Sample taken after 0 min: Small triangle line▲; Sample taken after 5 min: Small cube line■; Sample taken after 20 min: Big triangle line▲; Sample taken after 40 min: Dashed line -; Sample taken after 60 min: Big cube line■; Sample taken after 90 Circle line●.

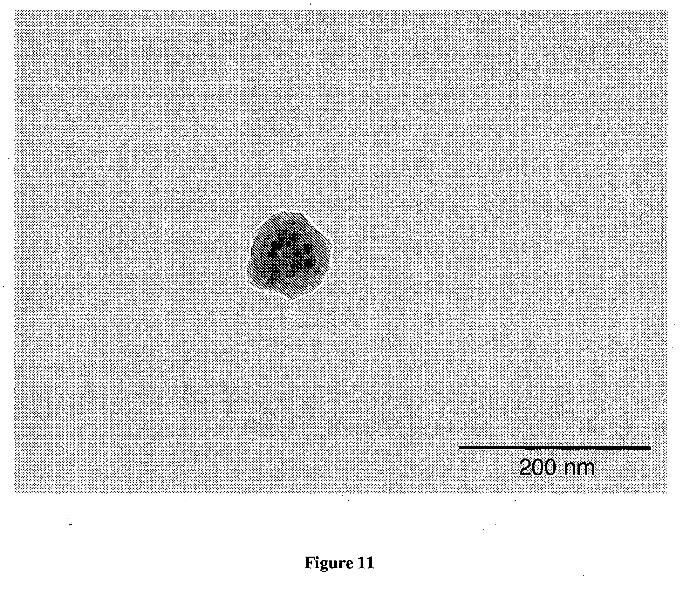

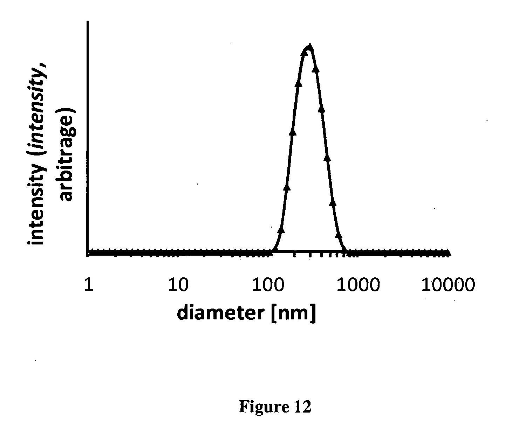

Example 7. Example 1 as above was carried out with the following specific conditions. The results are shown in figure 11 and 12.

3.6 nmol iron oxide nanoparticles (PI-DETA coated), 2.4 mg PI-PEO (M = 13500 g/mol), 0.6 mL of THF, 45 µL 1-pentanol, 30 mL water, 45 µL µL vinyl benzene, 45 µL divinyl benzene 1 mg VA-044, were stirred for 5 h at 50 °C.

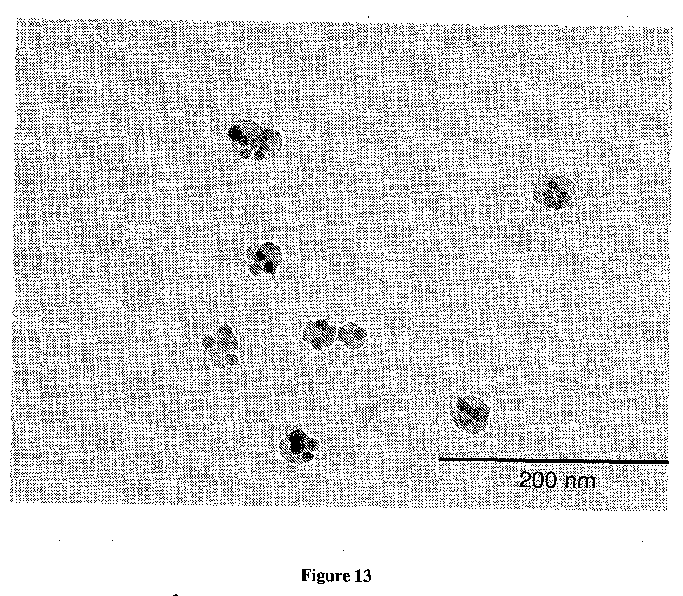

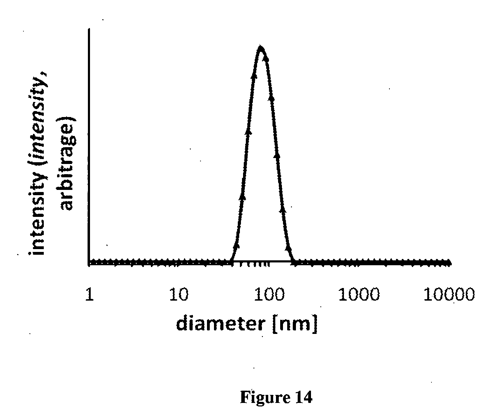

Example 8. Example 1 as above was carried out with the following specific conditions. The results are shown in figure 13 and 14.

2.4 nmol iron oxide nanoparticles (PI-DETA coated), 1.6 mg PI-PEO (M = 13500 g/mol), 1.6 mg AIBN, 0.4 mL of THF, 30 µL 1-pentanol, 30 mL water, 30 µL µL vinyl benzene, 30 µL divinyl benzene, were stirred for 5 h at 50 °C.

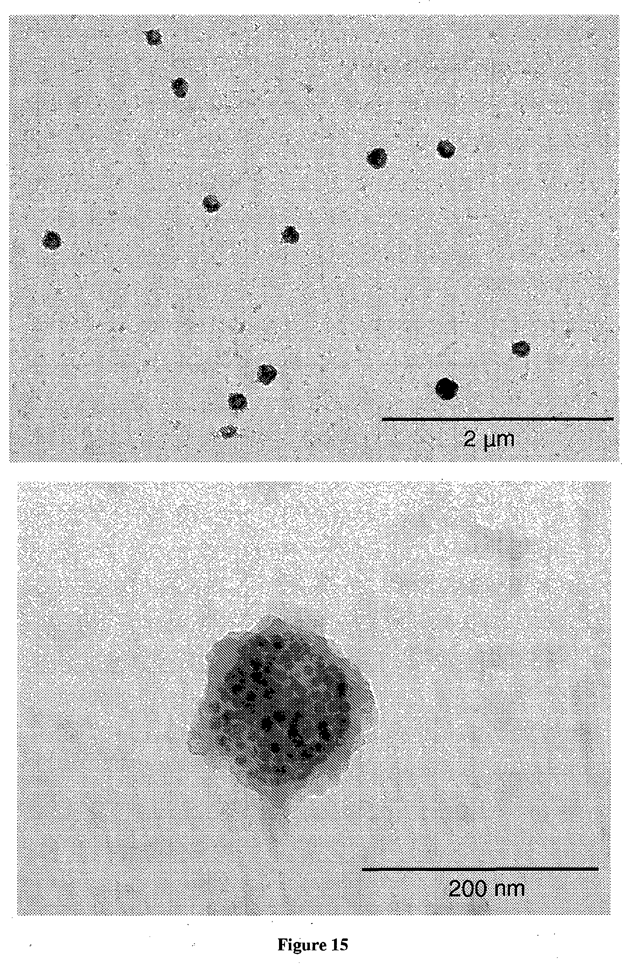

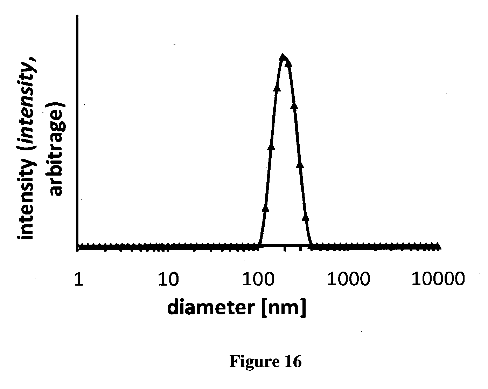

Example 9. Example 1 as above was carried out with the following specific conditions. The results are shown in figure 15 and 16.

2.5 nmol iron oxide nanoparticles (oleic acid coated), 68 mg N-(2-Methacryloyloxyethyl)-N,N-dimethyl-N-(3-sulfopropyl)ammoniumbetain, 4 mL of THF, 30 µL 1-pentanol, 30 mL water, 20 µL µL vinyl benzene, 20 µL divinyl benzene 1 mg VA-044, were stirred for 15 h at 50 °C.

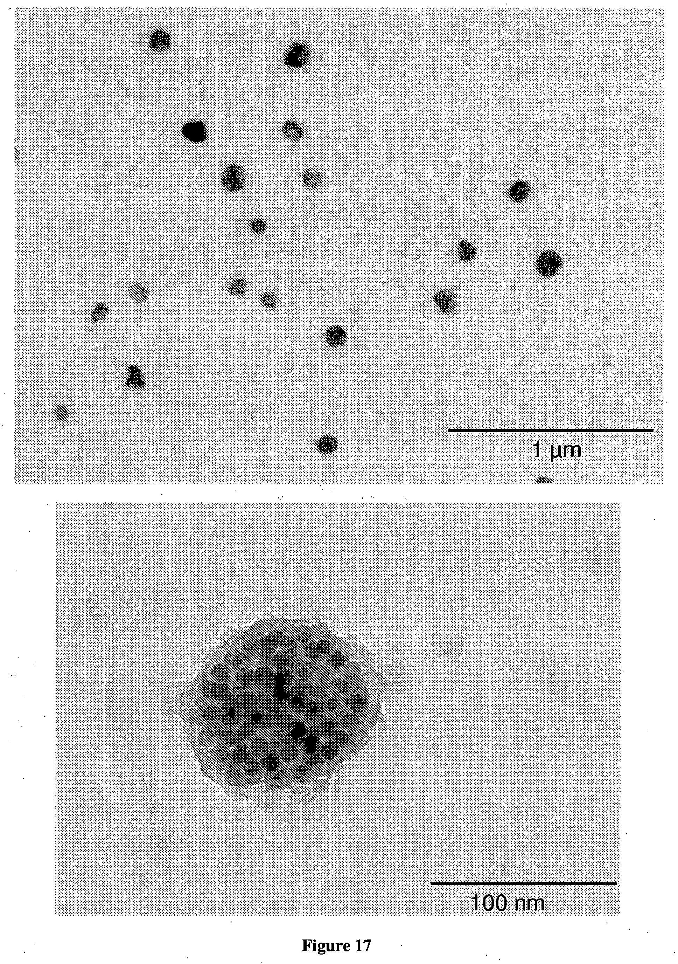

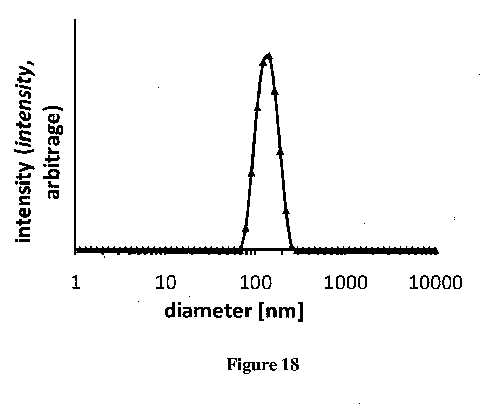

Example 10. Example 1 as above was carried out with the following specific conditions. The results are shown in figure 17 and 18.

2.5 nmol iron oxide nanoparticles (oleic acid coated), 68 mg polyethylene glycol monoacrylate (M = 336 g/mol), 4 mL of THF, 30 µL 1-pentanol, 30 mL water, 20 µL µL vinyl benzene, 20 µL divinyl benzene 1 mg VA-044, were stirred for 15 h at 50 °C.

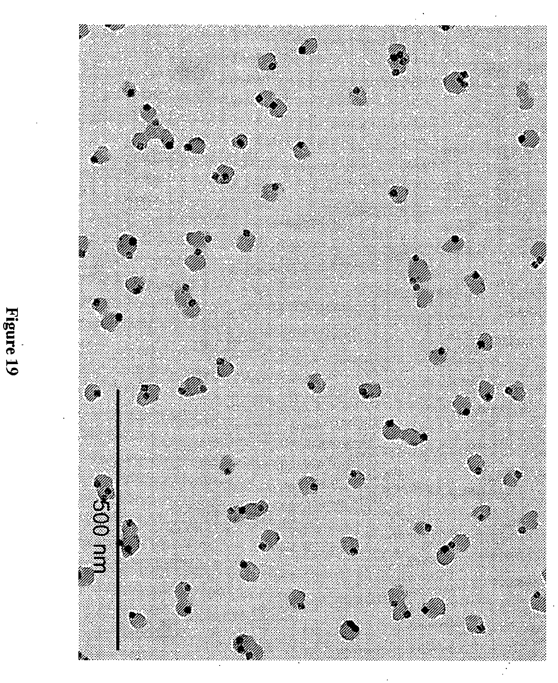

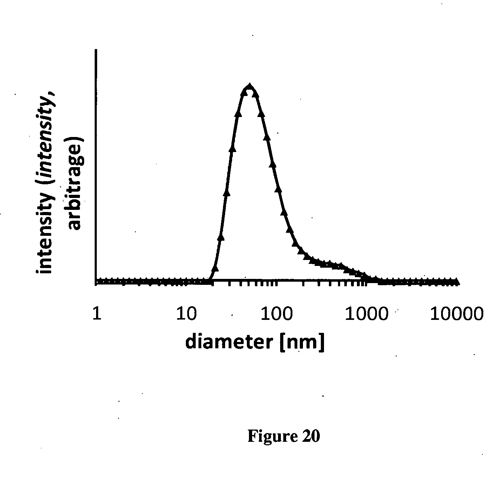

Example 11. Example 1 as above was carried out with the following specific conditions. The results are shown in figure 19 and 20.

1.1 nmol of iron oxide nanoparticles (oleic acid coated), 26 mg SDBS, 3 mL of THF, 30 µL 1-pentanol, 15 µL vinyl benzene, 15 µL divinyl benzene, 20 mg Dimethyl[3-[(2-methyl-l-oxoallyl)amino]propyl]-3-sulfopropylammoniumhydroxid ,20 mL water, 1 mg VA044, were stirred for 5 hours at 50 °C.

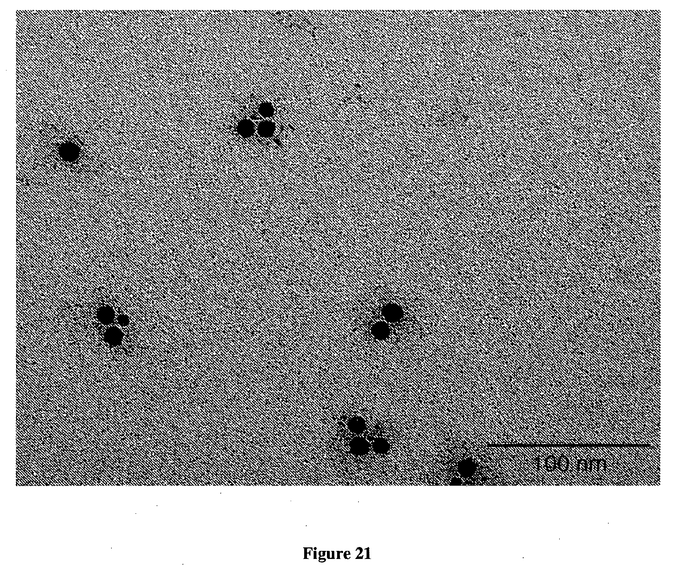

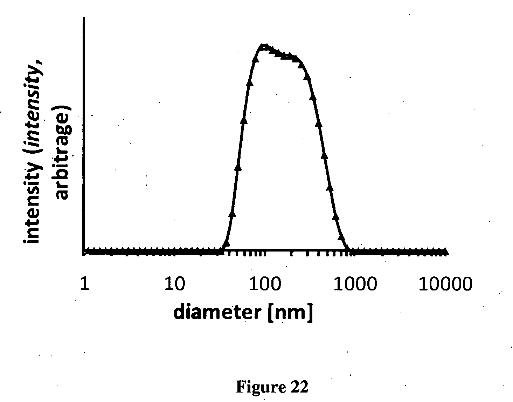

Example 12. Example 1 as above was carried out with the following specific conditions. The results are shown in figure 21 and 22.

3.6 nmol iron oxide nanoparticles (PI-DETA coated), 2.4 mg PI-PEO (M = 13500 g/mol), 0.6 mL of THF, 45 µL 1-pentanol, 20 mL water, 30 µL isoprene, 1 mg VA-044, were stirred for 72 h at 30 °C.

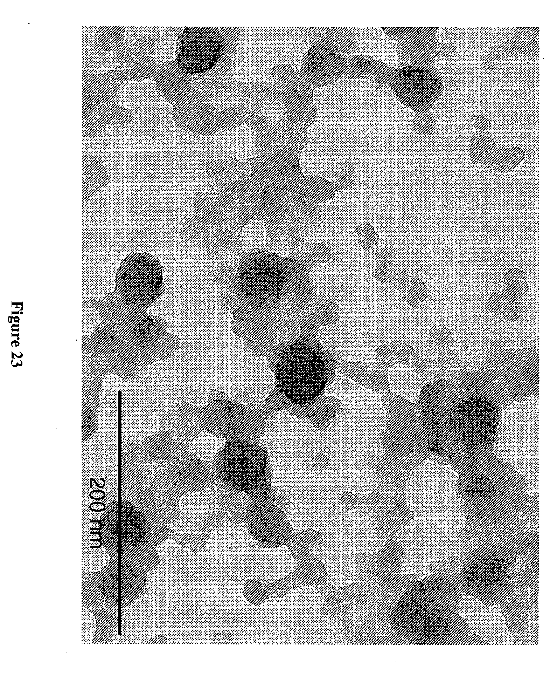

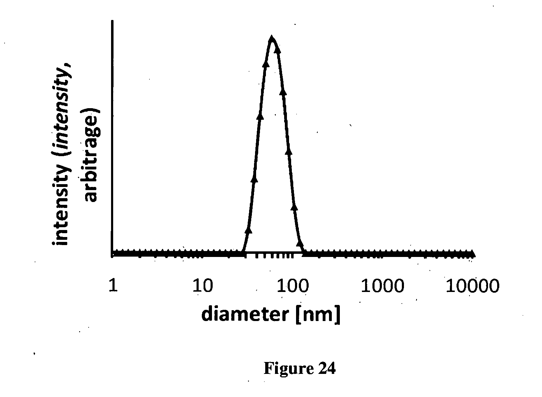

Example 13. Example 1 as above was carried out with the following specific conditions. The results are shown in figure 23 and 24.

4.2 nmol InP nanoparticles (tetradecanoic acid and octadecanoic acid coated), 10.3 mg PI-PEO (M = 3800 g/mol), 3 mL of THF, 30 µL 1-pentanol, 20 mL water, 22.5 µL vinyl benzene, 22.5 µL divinyl benzene, 1 mg VA-044, were stirred for 5 hours at 50 °C. The fluorescence properties were preserved.

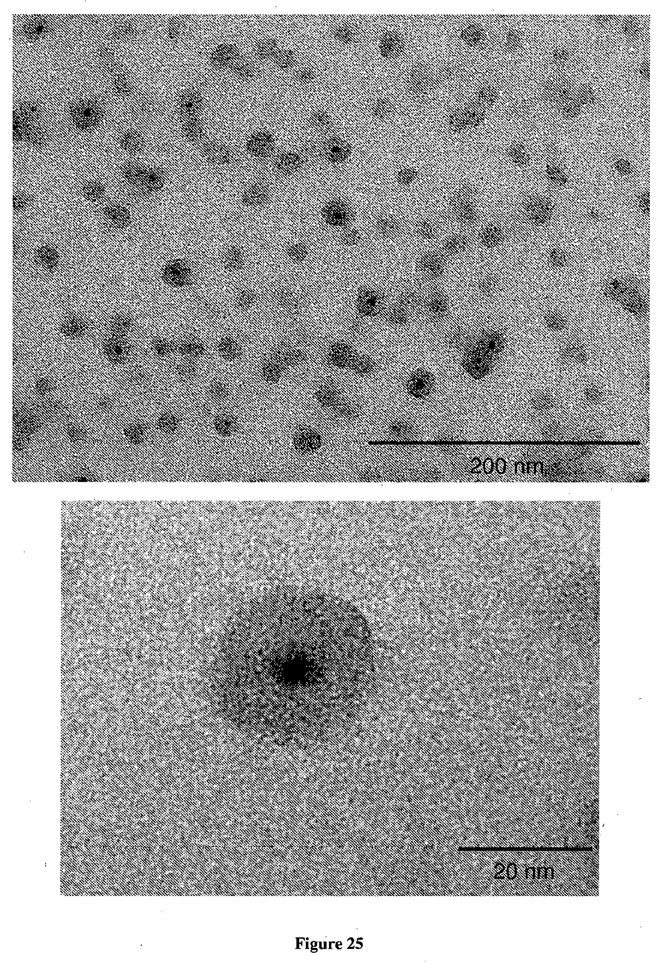

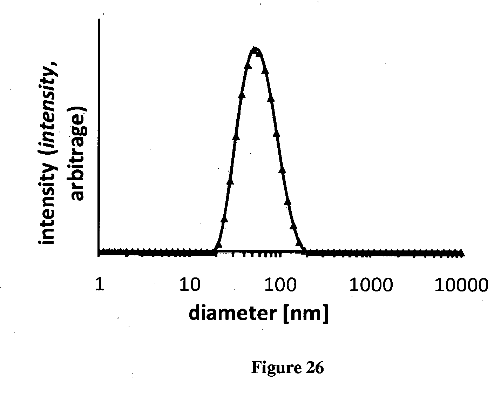

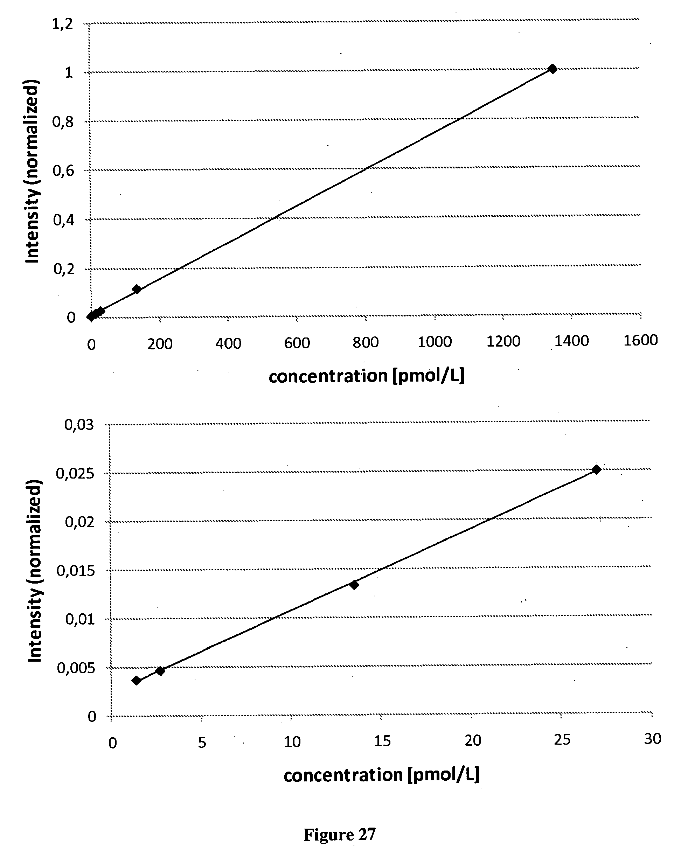

Example 14. Example 1 as above was carried out with the following specific conditions. The results are shown in figure 25, 26 and 27.

10 nmol CdSe/CdS/ZnS *core*/*shell*/*shell* quantum dot nanoparticles (PI-DETA coated), 10 mg PI-PEO (M = 13500 g/mol), 3 mL of THF, 30 µL 1-pentanol, 30 mL water, 15 µL vinyl benzene, 15 µL divinyl benzene, 1 mg VA-044, were stirred for 5 hours at 50°C. The fluorescence properties were maintained.

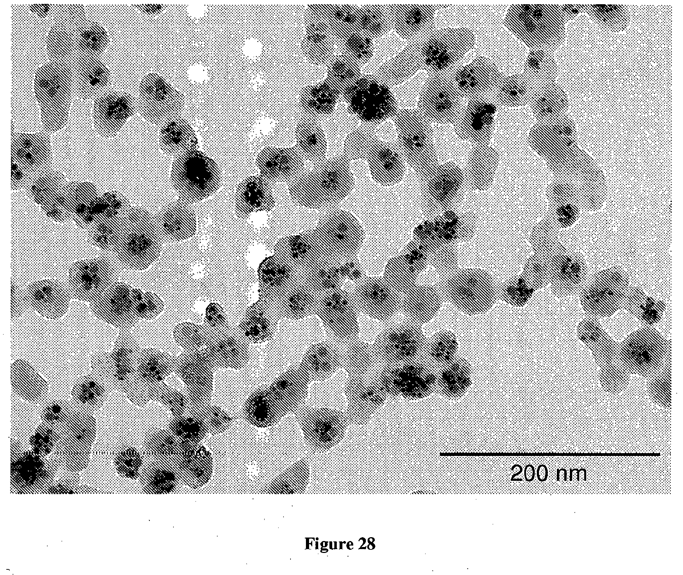

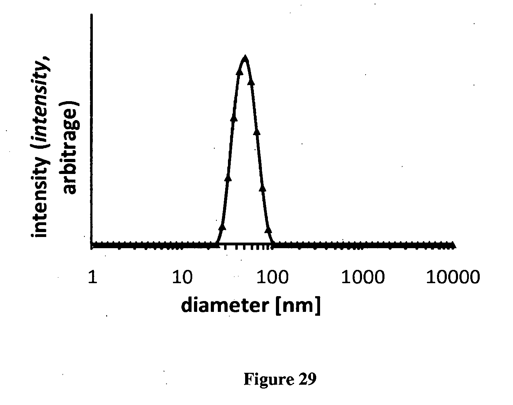

Example 15. Example 1 as above was carried out with the following specific conditions. The results are shown in figure 28 and 29.

0.5 nmol oxide nanoparticles (oleic acid coated), 0.5 nmol gold nanoparticles (oleylamine coated), 2.5 mg polysorbate 80, 0.5 mL of THF, 5 µL 1-pentanol, 2 mL water, 15 µL vinyl benzene, 5 µL divinyl benzene, 0.2 mg VA-044, were stirred for 5 hours at 44 °C.

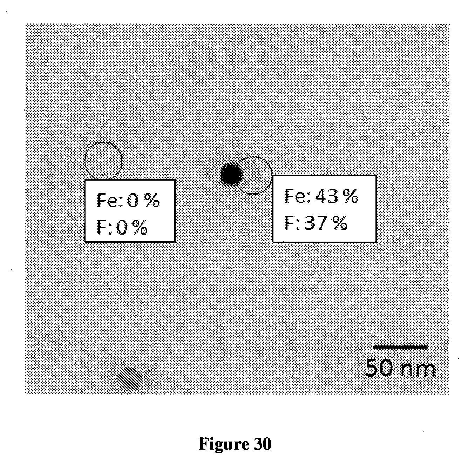

Example 16. Example 1 as above was carried out with the following specific conditions. The results are shown in figure 30.

156 pmol iron oxide nanoparticles (oleic acid coated), 76 mg polysorbate-80, 3 mL of THF, 5 µL 1-pentanol, 2 mL water, 25 µL 2,3,4,5,6-pentafluoro vinyl benzene and 10 µL divinyl benzene added, 2 mg VA-044, were stirred for 63 hours at 44 °C and afterward for 2 hours at 65 °C.

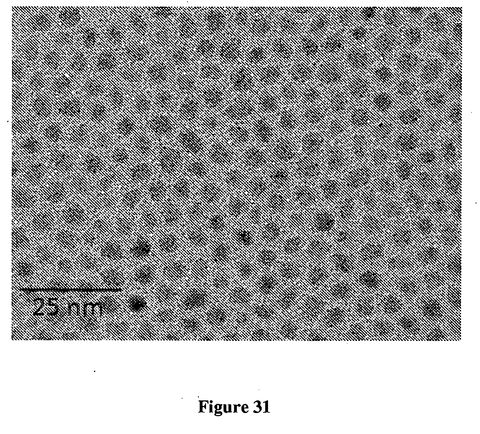

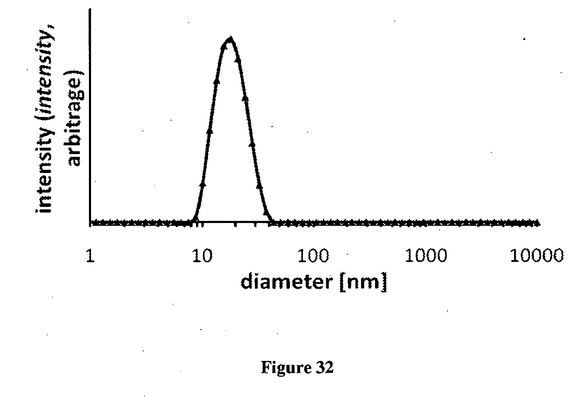

Example 17. Example 1 as above was carried out with the following specific conditions. The results are shown in figure 31 and 32.

0.39 mmol of CdSe/CdS/ZnS *core*/*shell*/*shell* quantum dot nanoparticles (TOP/TOPO and HDA coated), 0.79 mg polysorbate-80, 25 nL squalene, 0.3 mL THF, 0 µL 1-pentanol, 83 nL vinyl benzene, 17 nL divinyl benzene, 3 mL water, 0.5 mg AIBN, were stirred for 5 hours at 75 °C. The fluorescence properties were maintained.

Example 18. Encapsulation of iron oxide nanoparticles by seeded semi-batch emulsion polymerisation.

The encapsulation of iron oxide nanoparticles by seeded semi-batch emulsion polymerisation was carried out according to the following general conditions. 7.8 nmol to 33 nmol of iron oxide nanoparticles free of storage solvent was taken up in about 5 mL of THF. 4.97 to 6.73 g (3.79 to 5.14 mmol) of polysorbate-80 was added. The resultant mixture was homogenized for a few minutes by shaking gently to give a THF solution. The THF solution was then injected into 270 mL of water and heated to 70 °C. To start the reaction, about 150 mg (0.63 mmol) of sodium persulfate (NaPS) in 1 mL of water or about 250 mg (0.67 mmol) of lauryl peroxide (LPO) in 1 g of monomer were added at once. After the onset of the reaction, 10 g (99.9 mmol) of methyl methacrylate (MMA) or 10 g (78.0 mmol) of butyl acrylate (BA) were added steadily over one hour using a syringe pump. Samples were taken every 10 minutes in vials with hydroquinone monomethyl ether (MEHQ) to stop the reaction. Finally the dispersion was stirred for 1 hour at a temperature of 70 °C and afterwards the final sample was taken.

Example 19. Example 18 as above was carried out using 7.8 nmol of iron oxide nanoparticles, 10 g (99.9 mmol) of MMA as a monomer and 149 mg (0.63 mmol) of NaPS solvated in 1 mL of water as an initiator.

Example 20. Example 18 as above was carried out using 8.1 nmol of iron oxide nanoparticles, 10 g (99.9 mmol) of MMA as a monomer and 236 mg (0.64 mmol) of LPO solvated in 1 g (9.99 mmol) of MMA as an initiator.

Example 21. Example 18 as above was carried out using 33 nmol of iron oxide nanoparticles, 10 g (78.0 mmol) of BA as a monomer and a mixture of 77 mg of NaPS and 120 mg of LPO (0.325 mmol each), solvated in 1 mL of water and 1 g (7.8 mmol) of monomer, respectively.

The products were analysed by various analytical techniques.

Dynamic light scattering (DLS) analysis was used to measure the hydrodynamic diameter of the products. DLS was performed using a Malvern Zetasizer Nano ZS system with a helium-neon laser (λ = 633 nm). The product solutions were analysed in quartz cells. The product solutions were analysed at least twelve times for a period of ten seconds. The results of the DLS analysis were evaluated using a Dispersion Technology Software program (DTS) Version 5.1 by Malvern instruments.

Transmission electron microscopy (TEM) analysis was performed using a JEM-1011 made by Jeol (100 keV) and using a CM-300-UT made by Phillips (300 keV). The samples were prepared by providing a sample solution on a carbon film on a copper lattice, which was dried in prior to analysis.

Energy dispersive X-ray spectroscopy (EDX) analysis was performed using a TEM-type CM-300-UT spectrometer made by Philips. The sample was prepared in a same manner as the samples prepared in the analysis using TEM analysis.

UV-Vis was performed using a UV-Vis absorption spectrometer (Varian Cary 50) in the range of 300-800nm. Fluorescence spectroscopy was performed using a fluorescence emission spectrometer (Varian Eclipse) in the range 350-700nm. Samples of approximately 3 mL in quartz cells were analysed. A standard of Rhodamine 6G (in ethanol) was used to determine the fluorescence quantum yield. Any background signals were determined by measuring the respective pure solvent used to prepare the sample solution.

Having thus described the present invention in detail, it is to be understood that the foregoing detailed description of the invention is not intended to limit the scope of the invention thereof. The person skilled in the art will recognise that the invention can be practiced with modification within the scope of the attached claims. At least, it should be noted that the invention is not limited to the detailed description of the invention and/or of the examples of the invention.

### Reference Number List

- 10: Inorganic nanoparticle
- 20: Surfactant
- 21: Surfactant with functional group or affinity molecule
- 22: Surfactant with linker and functional group or affinity molecule
- 25: Hydrophobic ligand on the nanoparticle surface
- 26: Hydrophobic ligand with functional group or affinity molecule
- 27: Hydrophobic ligand with linker and functional group or affinity molecule
- 30: Hydrophilic moiety of surfactant
- 40: Hydrophobic moiety of surfactant
- 45: Functional group or affinity molecule
- 46: Linker
- 60: Polymerisable monomer
- 61: Monomer unit with functional group or affinity molecule
- 62: Monomer unit with linker and functional group or affinity molecule
- 65: Polymer chain
- 66: Polymer chain with functional group or affinity molecule
- 67: Polymer chain with linker and functional group or affinity molecule
- 70: Polymerisation initiator
- 90: Micellular combination
- 91: Micellular combination with functional group or affinity molecule
- 92: Micellular combination with linker and functional group or affinity molecule

## Claims

1. A method for the manufacture of a micellular combination (90) comprising the steps of:
- preparing a solution of a surfactant (20) with a hydrophobic moiety (40) and a hydrophilic moiety (30) and inorganic nanoparticles (10) surrounded in a core of hydrophobic ligands (25) in water-miscible solvent;
- transferring the solution of surfactant (20) and inorganic nanoparticles (10) into water
- adding a polymerisable monomer (60) to the aqueous phase
- adding water soluble polymerisation initiator (70) to start the emulsion polymerisation reaction.

2. The method of claim 1, wherein the inorganic nanoparticle (10) is any one of metal oxide like iron oxide, metal nanoparticles like Au, Ag, Pt, rare earth doped nanoparticle or a semiconducting nanoparticle including quantum dots.

3. The method of any one of the above claims, wherein the inorganic nanoparticles are a mixture of different inorganic nanoparticles (10) in the core.

4. The method of any one of the above claims, wherein the surfactant (20) is an amphiphilic block copolymer, and wherein the amphiphilic block copolymer preferably comprises polymerisable units in the hydrophobic moiety.

5. The method of anyone of claims 1 to 4, wherein a hydrophobic polymersation initiator (70) is added to the solution of inorganic nanoparticle (10) and surfactant (20).

6. The method of anyone of claims 1 to 5, wherein additionally a hydrophobic filler is added to the solution of inorganic nanoparticle (10) and surfactant (20).

7. The method of anyone of claims 1 to 6, wherein only the polymerisable monomer (60) polymerises to form a polymeric shell.

8. The method of anyone of claims 1 to 6, wherein the surfactant (20) co-polymerises with the polymerisable monomer (60) to form a polymeric shell.

9. The method of claim 8, wherein the hydrophobic ligands (25) additionally co-polymerise to form the polymeric shell.

10. The method of anyone of claims 1 to 9, wherein the surfactant (20) is polysorbate-80 or b-polyisoprene-b-polyethylene oxide.

11. The method of any of the above claims, wherein the polymerisable monomer (60) is at least one of, acrylate methacrylate, vinylacetate, vinyl benzene, divinyl benzene or fluorinated derivatives thereof.

12. The method of any of the above claims, wherein at least one of, the hydrophilic moiety (30), the polymerisable monomer (60), a polymer chain (65) of polymerisable monomers (60) and the hydrophobic ligand (25) comprise a functional group and/or affinity molecule, and wherein the functional group is preferably selected from the group comprising hydroxy, carboxyl, amine, ammonium, N-oxide, amide, imine, aldimine, ketal, acetal, ester, ether, disulfide, thiol, sulfonate, sulphate, sulphonamide, dithiocarbamate, phosphine, phosphine oxide, phosphate, phosphonate, silicate, silyl, borate, boronate, epoxy, azido, propargyloxy, halogen, nitro, isocyanate, isothiocyanate, carbodiimide, nitrile, isonitrile, hydrazone, oxime, and carbonyl, e.g. aldehyde groups, and/or wherein the affinity molecule is preferably of synthetic or biologic origin selected from the group comprising small druglike molecules, peptides, proteins, antibodies or fragments thereof, mono-, oligo-, or polysaccharides, nucleosides or nucleotide sequences or aptamers.

## Patentansprüche

1. Verfahren zur Herstellung einer Kombination vom Mizellen-Typ (90), das die folgenden Schritte umfasst:
- Herstellung einer Lösung eines Tensids (20) mit einer hydrophoben Einheit (40) und einer hydrophilen Einheit (30) sowie anorganischer Nanopartikel (10), umgeben in einem Kern von hydrophoben Liganden (25) in einem wassermischbaren Lösungsmittel;
- Überführen der Lösung des Tensids (20) und der anorganischen Nanopartikel (10) in Wasser
- Zugabe eines polymerisierbaren Monomers (60) zur wässrigen Phase
- Zugabe eines wasserlöslichen Polymersationsinitiators (70), um die Emulsionspolymerisationsreaktion zu starten.

2. Verfahren gemäß Anspruch 1, worin der anorganische Nanopartikel (10) ein beliebiger aus Metalloxid wie Eisenoxid, Metall-Nanopartikeln wie Au, Ag, Pt, Seltenerd-dotierten Nanopartikeln oder halbleitenden Nanopartikeln einschließlich Quantum-Dots ist.

3. Verfahren gemäß irgendeinem der obigen Ansprüche, worin die anorganischen Nanopartikel eine Mischung verschiedener anorganischer Nanopartikel (10) im Kern ist.

4. Verfahren gemäß irgendeinem der obigen Ansprüche, worin das Tensid (20) ein amphiphiles Block-Copolymer ist, und worin das amphiphile Block-Copolymer vorzugsweise polymerisierbare Einheiten in der hydrophoben Einheit aufweist.

5. Verfahren gemäß irgendeinem der Ansprüche 1 bis 4, worin ein hydrophober Polymerisationsinitiator (70) zur Lösung der anorganischen Nanopartikel (10) und des Tensids (20) hinzugegeben wird.

6. Verfahren gemäß irgendeinem der Ansprüche 1 bis 5, worin weiterhin ein hydrophober Füllstoff zur Lösung der anorganischen Nanopartikel (10) und des Tensids (20) hinzugegeben wird.

7. Verfahren gemäß irgendeinem der Ansprüche 1 bis 6, worin nur das polymerisierbare Monomer (60) polymerisiert, um eine polymere Hülle zu bilden.

8. Verfahren gemäß irgendeinem der Ansprüche 1 bis 6, worin das Tensid (20) mit dem polymerisierbaren Monomer (60) co-polymerisiert, um eine polymere Hülle zu bilden.

9. Verfahren gemäß Anspruch 8, worin die hydrophoben Liganden (25) zusätzlich co-polymerisieren, um die polymere Hülle zu bilden.

10. Verfahren gemäß irgendeinem der Ansprüche 1 bis 9, worin das Tensid (20) Polysorbat-80 oder b-Polyisopren-b-polyethylenoxid ist.

11. Verfahren gemäß irgendeinem der obigen Ansprüche, worin das polymerisierbare Monomer (60) mindestens eines aus Acrylat, Methacrylat, Vinylacetat, Vinylbenzol, Divinylbenzol oder fluorierten Derivaten davon ist.

12. Verfahren gemäß irgendeinem der obigen Ansprüche, worin mindestens eines aus der hydrophoben Einheit (30), dem polymerisierbaren Monomer (60), einer Polymerkette (65) des polymerisierbaren Monomers (60) und des hydrophoben Liganden (25) eine funktionelle Gruppe und/oder ein Affinitätsmolekül umfasst, und worin die funktionelle Gruppe vorzugsweise ausgewählt ist aus der Gruppe, bestehend aus Hydroxy, Carboxyl, Amin, Ammonium, N-Oxid, Amid, Imin, Aldimin, Ketal, Acetal, Ester, Ether, Disulfid, Thiol, Sulfonat, Sulfat, Sulphonamid, Dithiocarbamat, Phosphin, Phosphinoxid, Phosphat, Phosphonat, Silikat, Silyl, Borat, Boronat, Epoxy, Azido, Propargyloxy, Halogen, Nitro, Isocyanat, Isothiocyanat, Carbodiimid, Nitril, Isonitril, Hydrazon, Oxim und Carbonyl, beispielsweise Aldehydgruppen, und/oder worin das Affinitätsmolekül vorzugweise synthetischen oder biologischen Ursprungs ist, ausgewählt aus der Gruppe, bestehend aus kleinen arzneiartigen Molekülen, Peptiden, Proteinen, Antikörpern oder Fragmenten davon, Mono-, Oligo- oder Polysacchariden, Nukleosiden oder Nukleotidsequenzen oder Aptameren.

## Revendications

1. Procédé de fabrication d'une combinaison micellaire (90) comprenant les étapes consistant à :
- préparer une solution d'un tensioactif (20) comprenant une fraction hydrophobe (40) et une fraction hydrophile (30) et de nanoparticules inorganiques (10) entourées dans un coeur de ligands hydrophobes (25) dans un solvant miscible à l'eau ;
- transférer la solution de tensioactif (20) et de nanoparticules inorganiques (10) dans de l'eau ;
- ajouter un monomère polymérisable (60) à la phase aqueuse ; et
- ajouter un initiateur de polymérisation soluble dans l'eau (70) pour initier la réaction de polymérisation en émulsion.

2. Procédé selon la revendication 1, dans lequel la nanoparticule inorganique (10) est l'une quelconque d'un oxyde métallique comme l'oxyde de fer, de nanoparticules de métaux comme Au, Ag, Pt, de nanoparticules dopées aux terres rares ou d'une nanoparticule semiconductrice comprenant des points quantiques.

3. Procédé selon l'une quelconque des revendications ci-dessus, dans lequel les nanoparticules inorganiques sont un mélange de différentes nanoparticules inorganiques (10) dans le coeur.

4. Procédé selon l'une quelconque des revendications ci-dessus, dans lequel le tensioactif (20) est un copolymère séquencé amphiphile et dans lequel le copolymère séquencé amphiphile comprend de préférence des motifs polymérisables dans la fraction hydrophobe.

5. Procédé selon l'une quelconque des revendications 1 à 4, dans lequel un initiateur de polymérisation hydrophobe (70) est ajouté à la solution de nanoparticules inorganiques (10) et de tensioactif (20).

6. Procédé selon l'une quelconque des revendications 1 à 5, dans lequel une charge hydrophobe est également ajoutée à la solution de nanoparticules inorganiques (10) et de tensioactif (20).

7. Procédé selon l'une quelconque des revendications 1 à 6, dans lequel seul le monomère polymérisable (60) est polymérisé pour former une enveloppe polymère.

8. Procédé selon l'une quelconque des revendications 1 à 6, dans lequel le tensioactif (20) est copolymérisé avec le monomère polymérisable (60) pour former une enveloppe polymère.

9. Procédé selon la revendication 8, dans lequel les ligands hydrophobes (25) sont également copolymérisés pour former l'enveloppe polymère.

10. Procédé selon l'une quelconque des revendications 1 à 9, dans lequel le tensioactif (20) est le polysorbate-80 ou l'oxyde de b-polyisoprène-b-polyéthylène.

11. Procédé selon l'une quelconque des revendications ci-dessus, dans lequel le monomère polymérisable (60) est au moins un monomère parmi un acrylate, un méthacrylate, un acétate de vinyle, un vinylbenzène, un divinylbenzène ou leurs dérivés fluorés.

12. Procédé selon l'une quelconque des revendications ci-dessus, dans lequel au moins l'un parmi la fraction hydrophile (30), le monomère polymérisable (60), une chaîne polymère (65) de monomères polymérisables (60) et le ligand hydrophobe (25) comprennent un groupement fonctionnel et/ou une molécule d'affinité, et dans lequel le groupement fonctionnel est de préférence choisi dans le groupe constitué des groupements hydroxy, carboxyle, amine, ammonium, N-oxyde, amide, imine, aldimine, cétal, acétal, ester, éther, disulfure, thiol, sulfonate, sulfate, sulfonamide, dithiocarbamate, phosphine, oxyde de phosphine, phosphate, phosphonate, silicate, silyle, borate, boronate, époxy, azido, propargyloxy, halogène, nitro, isocyanate, isothiocyanate, carbodiimide, nitrile, isonitrile, hydrazone, oxime et carbonyle, par exemple des groupements aldéhyde, et/ou dans lequel la molécule d'affinité est de préférence d'origine synthétique ou biologique choisie dans le groupe comprenant de petites molécules, des peptides, des protéines, des anticorps ou des fragments de ceux-ci, des mono-, oligo- ou polysaccharides, des nucléosides ou des séquences nucléotidiques ou aptamères de qualité médicamenteuse.
